# EUROPEAN PATENT SPECIFICATION

(11) **EP 1 224 324 B1**
(45) Date of publication and mention of the grant of the patent: **11.05.2005**
(21) Application number: 00958835.1
(22) Date of filing: 08.09.2000
(51) Int. Cl.: C12Q 1/68, G01N 33/50, C12N 15/10

(54) **Two-hybrid screening method employing the bacterial transcriptional activator sigma-54**
Two-hybrid-Screeningverfahren unter Verwendung des bakteriellen Transcriptionsaktivators sigma-54
Méthode de criblage par double-hybride employant l'activateur transcriptionnel bactérien sigma-54

(30) Priority: 08.09.1999 GB 9921155
(43) Date of publication of application: 24.07.2002
(73) Proprietor: MEDICAL RESEARCH COUNCIL, London W1N 4AL (GB)
(72) Inventor: HOLLIGER, Philipp, Cambridge CB2 2QH (GB); SEPP, Tiina, Cambridge CB1 8YU (GB)
(74) Representative: Maschio, Antonio, Dr.
(86) International application number: PCT/GB2000/003450
(87) International publication number: WO 2001/018244

(56) References cited:
- WO-A-98/25947
- WO-A-99/28746

## Description

The present invention relates to a screening system useful for screening repertoires of DNA binding domains. In particular the invention relates to a screening system based on transcriptional activators of bacterial σ54-dependent promoters.

The majority of proteins involved in cellular functions do so by interacting with other proteins or nucleic acid sequences within the cell. Several approaches have been described that allow the *in vivo* selection of nucleic acids which express polypeptides capable of binding to proteins or DNA in the cell. Arguably the most powerful approaches are the yeast one- and two hybrid systems (Fields S. & Song O. (1989) *Nature* 340, 245; see US Patent 5,283,173) for the screening of protein-DNA and protein-protein interactions, respectively. However, the two-hybrid system requires an eukaryotic host and consequently the diversity that can be screened is limited. Furthermore the system notoriously suffers from an abundance of false positives.

Larger molecular repertoires can be prepared in bacterial hosts and a number of bacterial systems for the screening of protein-protein and protein-DNA interactions have also been reported. Two systems have been put forward in which the polypeptide chain of an enzyme is expressed in two parts fused to two candidate polypeptides. and in which interaction between the candidate polypeptides reconstitutes the function of the enzyme (Karimova G. et al (1998) *Proc. Nat. Acad. Sci USA* 95, 5752; Pelletier J.N. et al (1998) *Proc. Nat. Acad. Sci USA* 95, 12141). WO 98 25947 describes a two hybrid system that can detect homo and heterodimeric protein interactions in E Coli and other cells.

Several in vivo screens for DNA-binding proteins have also been reported (reviewed in Mossing M.C., Bowie J.U. & Sauer R.T. (1991) *Methods Enzymol*. 208, 604; Elledge SJ. et al (1989) *Proc. Nat. Acad. Sci USA* 86, 3689). Each of these methods involves the blockage of a hybrid σ70 promoter by the DNA binding protein. Repression of the promoter either prevents the production of conditionally toxic gene or alleviates repression of an antibiotic gene by transcriptional interference. The transcriptional interference assay (Elledge *et al*.) has been used successfully in one case to select DNA binding proteins with altered specificity (Sera T. & Schultz P.G. (1996) *Proc. Nat. Acad. Sci USA* 93, 2920).

Another σ70-based system utilises recruitment of the polymerase to the promoter by way of a protein-protein interaction between a protein domain fused to the RNA polymerase α subunit and another fused to the lambda repressor bound immediately upstream of the RNA polymerase promoter binding site (Dove S.L.. Joung J.K.. Hochschild A. (1997), *Nature* 386, 627). By replacing the lambda repressor DNA binding domain with a library of Zn-finger domains, specific DNA binding Zn-finger domains were selected (Joung J.K., Ramm E.I.. Pabo C.O. (2000) *Proc Natl Acad Sci U S A*, 97, 7382)

The alternative holoenzyme form of bacterial RNA polymerase (RNAP) contains the σ 54 factor (σ 54-RNAP). As has been previously shown, this polymerase, in most cases, forms a closed complex with the promoter. Unlike σ70 promoters at which the RNA polymerase is bound in an active form and is largely controlled by repression, the σ54 RNA polymerase holoenzyme is transcriptionally incompetent and is unable to initiate transcription by itself. Initiation of transcription requires the presence of a transcriptional activator that catalyses the isomerisation of the closed promoter complex to an open one. Typically, activator proteins bind to a specific upstream activation sequence (UAS) located 80 to 200 bp upstream of the σ 54 core promoter. The function of the UAS is to tether the activator in the right position and to bring it in the vicinity of the promoter in order to increase the efficiency of interaction between the σ 54 RNAP and the activator. Transcriptional activators of σ54 dependent promoters have been called bacterial enhancers because their mechanism of activation is superficially similar to the activation of transcription by enhancer proteins in eukaryotes (Kustu S. et al (1991) *Trends Biochem Sci* 16, 397).

Conversion of the σ 54 RNAP into an active form is catalysed by the binding of an enhancer protein coupled to hydrolysis of ATP. This unusual mechanism accounts for the low level of background transcription and the enormous difference (10⁴-10⁵) between on and off states in the strongest σ54 promoters effected by a single factor. In comparison, activators of σ70 promoters such as CAP or λcI increase transcription levels usually by less than 10-fold.

Transcriptional activators of σ54 promoters (also known as enhancer-binding proteins or EBPs) share a common structure (see Morrett and Segovia. (1993) J. Bacteriol. 6067-6074) comprising a non-conserved N-terminal domain which has a putative regulatory function, a central domain which is responsible for transcriptional activation, and a C-terminal DNA binding domain which binds the relevant UAS in the target gene. The domains are modular: the central and N-terminal domains together are capable of constitutive activation of σ54 RNAP when overexpressed. At least in some cases, the isolated DNA binding domain is capable of specifically binding its DNA recognition site.

In many instances, interaction between σ54 RNAP and the activator is enhanced by a cellular factor which promotes DNA bending between the UAS and the σ54 promoter (Freundlich *et al*., (1992) Mol. Microbiol. 6:2557-2563). This factor, known as integration host factor (IHF) acts to promote transcription from σ54 promoters.

### Summary of the Invention

We provide herein a novel screening system which is based on transcriptional activators of σ54-based promoters.

According to a first aspect of the invention, therefore, there is provided a method for detecting a protein-nucleic acid interaction between a nucleic acid molecule and a protein molecule, in vitro or in vivo in bacteria, comprising the steps of:
a) providing one or more hybrid σ54 activator proteins comprising a heterologous nucleic acid binding domain and a constitutively active σ54 transcription activating domain;
b) providing one or more nucleic acid molecules comprising a binding site for the nucleic acid binding domain and a binding site for σ54 RNAP. which directs the expression of a reporter gene and leads to upregulation thereof in response to activation by the σ54 transcription activating domain; and
c) detecting expression of the reporter gene.

The invention provides a reporter system which is characterised by very low levels of background expression, since the σ54 polymerase is transcriptionally incompetent in the absence of a σ54 transcriptional activator. Since, at physiological concentrations, the binding of the transcriptional activator to the nucleic acid is required in order to activate transcription by σ54 RNAP, the system of the invention may be used as a tool for investigating and/or screening protein/nucleic acid interactions exploiting the reporter gene read-out.

In the first aspect of the invention, either the nucleic acid binding protein or the nucleic acid molecule may be provided in the form of a repertoire of molecules. Repertoires of hybrid σ54 activator proteins preferably are partially or completely randomised at least in the heterologous nucleic acid binding domain. This allows selection from the library of molecules having desired nucleic acid binding characteristics.

Repertoires of nucleic acid molecules advantageously are partially or completely randomised in the binding site for the nucleic acid binding domain of the σ54 activator protein. This allows selection of nucleic acid molecules having desired binding sites for the chimeric activators.

In a second aspect of the invention. there is provided a system for selecting protein-protein interactions based on the constitutively active hybrid σ54 activators described above. The system according to the invention is conceptually similar to the yeast two-hybrid system.

Accordingly, there is provided a method for detecting a protein-protein interaction in vitro or vivo in bacteria, comprising the steps of:
a) providing a first hybrid protein comprising a nucleic acid binding domain and a first polypeptide sequence bait;
b) providing a second hybrid protein comprising a prey polypeptide sequence and constitutively active σ54 transcription activating domain;
c) providing a nucleic acid molecule comprising a binding site for the nucleic acid binding domain and binding site for σ54 RNAP which directs the expression of a reporter gene and leads to upregulation thereof in response to activation by the σ54 transcription activating domain;
d) incubating the first and second hybrid proteins together with the nucleic acid molecule such that the prey and bait polypeptide sequences may bind, thereby forming a hybrid protein comprising both a nucleic acid binding domain and a σ54 transcription activating domain; and
e) detecting expression of the reporter gene.

As will be apparent to those skilled in the art, reference to a "binding site" for the nucleic acid binding domain includes the provision of several appropriately spaced binding sites in the nucleic acid molecule.

As with the yeast two-hybrid system, in which a modular transcription factor is assembled though binding of DNA binding domain/bait and transcription activating domain/prey hybrids, the association of the nucleic acid binding domain and the σ54 transcription activating domain through the bait/prey interaction allows the detection of, and screening for, protein-protein binding interactions *in vivo* and *in vitro.* Advantageously, the bait and/or prey polypeptide sequences are provided in the form of repertoires, which may be partially or completely randomised. This allows selection of prey polypeptides based on their ability to form interactions with a desired bait (or vice versa). As the assay may be conducted *in vivo*, in a bacterium, the invention permits the detection of *in vivo* binding interactions between polypeptides in bacteria.

It will be apparent that the hybrid proteins useful in the methods of the invention are advantageously provided in the form of nucleic acid vectors or libraries thereof capable of expressing said proteins in a host bacterium. Advantageously, the vector(s) include first and second chimeric genes which encode the hybrid proteins of the invention. Preferably, the vectors also include means for replication in bacteria. Also included may be one or more marker genes, the expression of which in the bacterium permits selection of cells containing the vector(s) from cells that do not contain the vector(s). Preferably, the vector(s) are plasmid(s).

In a third aspect, the invention provides a method for screening a repertoire of candidate DNA-bending polypeptides in vivo in bacteria, comprising the steps of:
a) providing a repertoire of candidate polypeptide factors with potential to induce bending of DNA;
b) providing a σ54 activator protein comprising a nucleic acid binding domain and a σ54 transcription activating domain;
c) providing a nucleic acid molecule comprising a binding site for the nucleic acid binding domain and binding site for σ54 RNAP which directs the expression of a reporter gene and leads to upregulation thereof in response to activation by the σ54 transcription activating domain;
d) incubating the repertoire and σ54 activator together with the nucleic acid molecule in a IHF (integration host factor) host cell, such that σ54 activator and the nucleic acid molecule may interact, and transcription activated from the σ54 KNAP binding site in a manner dependent on DNA bending by the polypeptide factors; and
e) detecting expression of the reporter gene.

It is known that activation by σ54 activators may be regulated by factors which induce DNA bending in the target gene. For example, the host factor IHF is known to potentiate σ54 activation; moreover, it may be replaced by alternative DNA bending polypeptides, or by intrinsically bent DNA.

The invention moreover provides methods for development of improved σ54 activator-based tools.

The first chimeric gene includes a nucleic acid sequence that encodes a nucleic acid-binding domain and a first (bait) test protein or protein fragment in such a manner that the first test protein is expressed as part of a hybrid protein with the nucleic acid-binding domain.

The second chimeric gene also includes a promoter and a transcription termination signal to direct transcription. The second chimeric gene moreover includes a nucleic acid sequence that encodes a σ54 transcriptional activation domain and a second (prey) test protein or protein fragment into the vector, in such a manner that the second test protein is capable of being expressed as part of a hybrid protein with the transcriptional activation domain.

The disclosure moreover provides kits for practising the invention as defined by the appended claims, which kits advantageously comprise a container, two vectors, and a host cell. The first vector contains a promoter and may include a transcription termination signal functionally associated with the first chimeric gene in order to direct the transcription of the first chimeric gene. The chimeric gene advantageously comprises one or more unique restriction site(s) to insert a nucleic acid sequence encoding a test bait polypeptide. The kit also may also include a second vector which contains a second chimeric gene, optionally comprising one or more unique restriction site(s) to insert a nucleic acid sequence encoding the prey polypeptide; alternatively, the second chimeric gene may be present on the same vector as the first chimeric gene.

### Brief description of the Figures

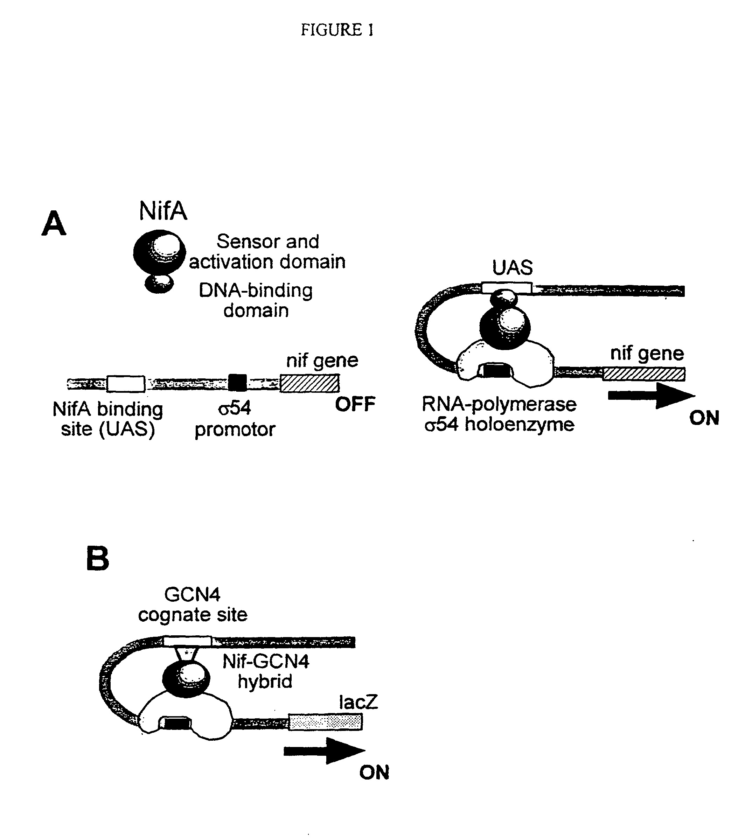

Figure 1A is a schematic representation of σ54 KNAP activation by σ54 activator NifA.
Figure 1B is a schematic representation of the invention, in which the σ54 DNA binding domain is replaced with a heterologous GCN4 DNA binding domain.

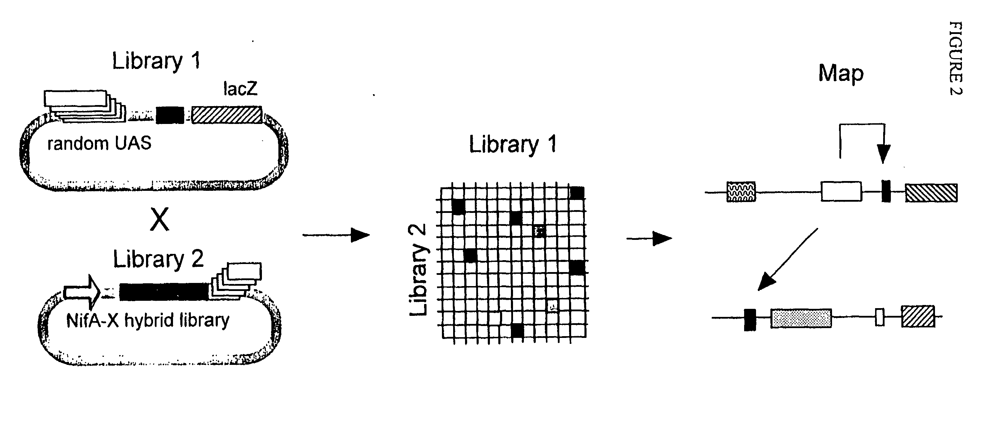

Figure 2 is a schematic representation of the first aspect of the present invention, in which a library of DNA binding domains is screened together with a library of DNA binding domain binding sites to identify protein:DNA binding pairs.
Figure 3 shows the activation of transcription by NifA-chimera as expressed as percent of wt activity (NifA/UAS). Nif-GCN4 (in presence of the NifAΔC coactivator (NifADC)) show close to wt activity. Equal activity is observed for the two distinct GCN$ DNA recognition sites (ATF/Creb and AP-1). Less than 1% wt activity is observed with a non-cognate reporter such as one bearing the wt nifH UAS.
Figure 4 shows the activation of transcription by NifA-chimera as expressed as percent of wt activity (NifA/UAS). Nif-ERDBD (in presence of the NifAΔC coactivator (NifADC)) shows ca. 80% of wt activity. Very little activation is observed with a non-cognate reporter bearing the DNA recognition site (GRE) for the closely related Glucocorticoid receptor.
Figure 5 shows the coactivation by different NifA variants as expressed as percent of wt activity (NifAwt). NifA from Klebsielta pneumoniae (NifAKp) is superior to al others, even exceeding wt activity (up to 160%). NifAKp with its DNA domain deleted (NifAΔCKp (NIfADCKp)). is almost as active.

### Detailed Description of the Invention

Unless defined otherwise, all technical and scientific terms used herein have the same meaning as commonly understood by one of ordinary skill in the art (e.g., in bacterial cell culture, molecular genetics, nucleic acid chemistry, protein chemistry and biochemistry). Standard techniques are used for molecular, genetic and biochemical methods (see generally, Sambrook *et al*., Molecular Cloning: A Laboratory Manual, 2d ed. (1989) Cold Spring Harbor Laboratory Press, Cold Spring Harbor, N.Y. and Ausubel *et al*., Short Protocols in Molecular Biology (1999) 4^{th} Ed, John Wiley & Sons, Inc.), chemical methods, pharmaceutical formulations and delivery and treatment of patients.

### A: Nucleic Acids and Proteins

As used herein, "nucleic acid" refers to any natural nucleic acid, including RNA and DNA as well as synthetic nucleic acid comprising modified or synthetic bases, and mixtures of modified or synthetic bases with natural bases. Such modified and/or synthetic bases may be referred to as derivatives of DNA or RNA. Preferably, "nucleic acid" refers to DNA.

The invention includes the use of modified and/or artificial "nucleic acids". A number of modifications have been described that alter the chemistry of the phosphodiester backbone, sugars or heterocyclic base components of nucleic acids.

Among useful changes in the backbone chemistry are phosphorothioates; phosphorodithioates, where both of the non-bridging oxygens are substituted with sulphur; phosphoroamidites: alkyl phosphotriesters and boranophosphates. Achiral phosphate derivatives include 3'-O'-5'-S-phosphorothioate, 3'-S-5'-O-phosphorothioate, 3'-CH2-5'-O-phosphonate and 3'-NH-5'-O-phosphoroamidate. Peptide nucleic acids replace the entire phosphodiester backbone with a peptide linkage.

Sugar modifications are also known. The α-anomer of deoxyribose may be used, where the base is inverted with respect to the natural β-anomer. The 2'-OH of the ribose sugar may be altered to form 2'-O-methyl or 2'-O-allyl sugars, which provides resistance to degradation without comprising affinity.

Modification of the heterocyclic bases must maintain proper base pairing. Some useful substitutions include deoxyuridine for deoxythymidine; 5-methyl-2'-deoxycytidine and 5-bromo-2'-deoxycytidine for deoxycytidine. 5-propynyl-2'-deoxyuridine and 5-propynyl-2'-deoxycytidine have been shown maintain biological activity when substituted for deoxythymidine and deoxycytidine, respectively.

As used herein, the term "protein" includes single-chain polypeptide molecules as well as multiple-polypeptide complexes where individual constituent polypeptides are linked by covalent or non-covalent means. As used herein, the terms "polypeptide" and "peptide" refer to a polymer in which the monomers are amino acids and are joined together through peptide or disulphide bonds. The term domain also refers to polypeptides and peptides having biological function. A peptide useful in the invention will have a binding or transcription activating capability, i.e., with respect to binding to nucleic acids, other proteins or polypeptides, and activation of σ54 RNAP transcription. It also may have another biological function that is a biological function of a protein or domain from which the peptide sequence is derived.

A hybrid protein is a protein or polypeptide which comprises constituent parts derived from at least two naturally-occurring or artiticial proteins. In particular, it may comprise the DNA-binding domain of one protein and the protein-binding or transcription activating domain of a second protein.

### B: σ54 Activators

Activators of σ54 transcription are well known and have been reviewed, for example, by Buck *et al*., J Bacteriol. 2000 Aug;182(15):4129-36; Studholme and Buck, FEMS Microbiol Lett. 2000 May 1;186(1):1-9; Shingler, Mol Microbiol, 1996 Feb;19(3):409-16; Goosen and van der Putte, Mol Microbiol. 1995 Apr;16(1):1-7; Merrick, Mol Microbiol. 1993 Dec:10(5):903-9; and others. A family of such activator proteins has been defined, and its members found to share homology in the central (catalytic) domain which is responsible for σ54 RNAP activation.

Members of the family include the following (the numbers are GenBank accession numbers)
dbj|BAA16379.1| (D90877) FORMATE HYDROGENLYASE TRANSCRIPTIONAL ACTIVATOR.
emb|C.AA26472-1| (X02616) pot. NifA gene product (aa 1-484) [Klebsiella pneumoniae]
emb|CAA53584.1| (X75972) anfA [Rhodobacter capsulatus]
emb|CAA92413.1| (Z68203) NifA homologue [Rhizobium sp.]
emb|CAA93242.1| (Z69251) MopR [Acinetobacter calcoaceticus]
emb|CAB53157.1| (X07567) NifA1 [Rhodobacter capsulatus]
emb|CAB56537.1| (AJ249642) response regulator [Pseudomonas stutzeri]
gb|AAA58220.1| (U 18997) ORF_o532 [Escherichia coli]
gb|AAA99303.1| (L43064) regulatory protein [Pseudomonas aeruginosa]
gb|AAB91397.1| (AF033203) NifAII protein [Rhodobacter capsulatus]
gb|AAC05586.1| (AF006075) regulatory protein [Bacillus subtilis]
gb|AAC37124.1| (L81176) FleQ [Pseudomonas aeruginosa]
gb|AAC45640.1|(AF010585) putative sigma 54 activator [Caulobacter crescentus]
gb|AAC46367.1|(AF014113) two-component response regulator [Vibrio cholerae]
gb|AAD34591.1|AF145956_1 (AF145956) transcriptional activator NifA [Rhodospirillum rubrum]
gb|AAD38416.1|(AF155934) NifA [Alcaligenes faecalis]
gb|AAF28395.1|(AF069392) FlaM [Vibrio parahaemolyticus]
gb|AAF33506.1|(AF170176) Salmonella typhimurium transcriptional regulatory protein gb|AAF61932.1|(AF230804) sigma-54 activator protein Act1 [Myxococcus xanthus]
gb|AAF85342.1|AE004061_7(AE004061) two-component system, regulatory protein [Xylella fastidiosa]
gb|AAF94676.1|(AE004230) sigma-54 dependent response regulator [Vibrio cholerae]
gb|AAF95280.1|(AE004286) sigma-54 dependent response regulator [Vibrio cholerae]
gb|AAF96095.1|(AE004358) sigma-54 dependent transcriptional regulator [Vibrio cholerae]
gb|AAG01527.1|AF288483_1 (AF288483) NifA [Azospirillum brasilense]
pir∥A48291 ornithine decarboxylase inhibitor - Escherichia coli
pir∥B49940 nitrogen regulator I homolog - Escherichia coli
pir∥C70320 transcription regulator NifA family - Aquifex aeolicus
pir∥C70396 transcription regulator NtrC family - Aquifex aeolicus
pir∥C70454 transcription regulator NtrC family - Aquifex aeolicus
pir∥D70315 transcription regulator NtrC family - Aquifex aeolicus
pir∥H69581 transcription activator of acetoin dehydrogenase operon acoR - Bacillus subtilis
pir∥I39719 nitrogen regulatory protein - Agrobacterium tumefaciens
pir∥JC5471 regulatory protein NifA - Azospirillum lipoferum
pir∥T08624 probable NtrC-type response regulator - Eubacterium acidaminophilum
sp|P03027|NIFA_KLEPN NIF-SPECIFIC REGULATORY PROTEIN
sp|P09570|NIFA_AZOVI NIF-SPECIFIC REGULATORY PROTEIN
sp|P12627|VNFA_AZOVI NITROGEN FIXATION PROTEIN VNFA
sp|P14375|HYDG_ECOLI TRANSCRIPTIONAL REGULATORY PROTEIN HYDG
sp|P21712|YFHA_ECOLI HYPOTHETICAL 49.1 KD PROTEIN IN GLNB-PURL INTERGENIC REGION (ORFXB)
sp|P24426|NIFA_RHILT NIF-SPECIFIC REGULATORY PROTEIN
sp|P25852|HYDG_SALTY TRANSCRIPTIONAL REGULATORY PROTEIN HYDG
sp|P27713|NIFA_HERSE NIF-SPECIFIC REGULATORY PROTEIN
sp|P30667|NIFA_AZOBR NIF-SPECIFIC REGULATORY PROTEIN
sp|P38035|RTCR_ECOLI TRANSCRIPTIONAL REGULATORY PROTEIN RTCR
sp|P54929|NIFA_AZOLI NIF-SPECIFIC REGULATORY PROTEIN
sp|P56266|NIFA_KLEOX NIF-SPECIFIC REGULATORY PROTEIN
sp|Q06065|ATOC_ECOLI ACETOACETATE METABOLISM REGULATORY
PROTEIN ATOC (ORNITHINE/ARGININE
sp|Q46802|YGEV_ECOLI HYPOTHETICAL SIGMA-54-DEPENDENT
TRANSCRIPTIONAL REGULATOR IN
sp|Q53206|NIFA_RHISN NIF-SPECIFIC REGULATORY PROTEIN
sp|Q9ZIB7|TYRR_ERWHE TRANSCRIPTIONAL REGULATORY PROTEIN TYRR

Moreover, a number of polypeptides belonging to the σ54 activator family have been described whose 3D structures are known. These include: 113161 acetoin catabolism regulatory protein; 113629 alginate biosynthesis transcriptional regulatory protein ALGB; 266789 type 4 fimbriae expression regulatory protein PILR; 113833 nitrogen fixation protein ANFA; 138884 nitrogen fixation protein VNFA; 128219 nif-specific regulatory protein; 3024194 nif-specific regulatory protein; acetoacetate metabolism regulatory protein ATOC 1168553 (ornithine/arginine decarboxylase inhibitor) (ornithine decarboxylase antizyme); 417166 transcriptional regulatory protein HYGD; 266622 nif-specific regulatory protein; 1352500 nif-specific regulatory protein; 128224 nif-specific regulatory protein; 128225 nif-specific regulatory protein; 128221 nif-specific regulatory protein; 128226 nif-specific regulatory protein; 1346014 transcriptional regulatory protein FLBD; 549560 hypothetical sigma-54-dependent transcriptional regulator in GUTQ-HYPF intergenic region; 139857 transcriptional regulatory protein XYLR (67 kd protein); 120053 formate hydrogenlyase transcriptional activator; 2507375 hypothetical 49.1 kd protein in GLNB-PURI intergenic region (ORFXB) (orf-2); 134961 signal-transduction and transcriptional-control protein; 1171795 nitrogen assimilation regulatory protein; 417388 nitrogen regulation protein nr(i); 123466 hydrogenase transcriptional regulatory protein HOXA; 399925 hydrogenase transcriptional regulatory protein HOXA; 585586 nitrogen assimilation regulatory protein NTRX; 118399 c4-dicarboxylate transport transcriptional regulatory protein DCTD: 585267 pathogenicity locus probable regulatory protein HRPR; 1346313 pathogenicity locus probable regulatory protein HRPS: 549447 pathogenicity locus probable regulatory protein WTSA: 585909 arginine utilization regulatory protein ROCR; 136600 transcriptional regulatory protein TYRR; 1174836 transcriptional regulatory protein TYRR homolog: 123748 hydrogenase transcriptional regulatory protein HUPR1; 128604 nitrogen regulation protein NTRC; 169293 glycerol metabolism operon regulatory protein; and 129957 phosphoglycerate transport system transcriptional regulatory protein PGTA . The numbers are GenBank gi numbers.

Preferably, the hybrid σ54 activator is based on the NifA activator. The Nif family of bacterial enhancers regulate expression of nitrogenase components from σS4 promoters in nitrogen-fixing bacteria, and are inhibited by NifL (Austin S. et al (1994) *J. Bacteriol*. 176, 3460). In bacteria lacking NifL, NifA is constitutively active. NifA is modular in architecture and it is shown herein that this allows for the swapping of the natural DNA-binding domain (DBD) for heterologous DBDs. Such NifA-DBD chimaeras are inactive on the wild type promoter, but activate transcription from hybrid promoters bearing their cognate target sequences.

Advantageously, the hybrid σ54 activator may be based on *E. coli* PspF (see Jovanovic *et al*., (1996) J. Bacteriol. 178:1936-1945). PspF lacks the N-terminal regulatory domain typical of σ54 activators, and is constitutively active but negatively regulated by PspA. Thus, in bacteria lacking PspA, PspF is constitutively active.

Other σ54 activators may be rendered constitutively active by removal of the N-terminal regulatory domain or by appropriate mutation.

Nucleic acid binding sequences or domains are known in the art and may be derived from σ54 activator proteins or any other DNA binding proteins, whether naturally-occurring or synthetic. Moreover, DNA-binding domains may be synthesised by partial or complete randomisation. Many naturally-occurring DNA-binding proteins contain independently folded domains for the recognition of DNA. and these domains in turn belong to a large number of structural families, such as the leucine zipper, the homeodomain. the "helix-turn-helix", the zinc finger and various other transcription factor families.

### C: Libraries

The term library refers to a mixture of heterogeneous polypeptides or nucleic acids. The library is composed of members, which have a unique polypeptide or nucleic acid sequence. To this extent. *library* is synonymous with *repertoire*, although in general the term "library" is used herein to denote the source of the repertoire - e.g. a library of nucleic acid molecules which encodes a repertoire of polypeptides. Sequence differences between library members are responsible for the diversity present in the library. The library may take the form of a simple mixture of polypeptides or nucleic acids, or may be in the form organisms or cells, for example bacteria, viruses, animal or plant cells and the like, transformed with a library of nucleic acids. Advantageously, the nucleic acids are incorporated into expression vectors, in order to allow expression of the polypeptides encoded by the nucleic acids. In a preferred aspect, therefore, a library may take the form of a population of host organisms, each organism containing one or more copies of an expression vector containing a single member of the library in nucleic acid form which can be expressed to produce its corresponding polypeptide member. Thus, the population of host organisms has the potential to encode a large repertoire of genetically diverse polypeptide variants.

Libraries of hybrid proteins may be prepared and selected together with libraries of hybrid nucleic acids. "Crossing" of hybrid libraries is performed by combinatorial infection, which has been employed successfully to generate very large antibody libraries (Griffiths et al (1994) EMBO J. 13,3245).

Although libraries for use in the present invention may be phage libraries, as is known in the art, it is possible to use alternative libraries which are constructed using other vectors, such as plasmids. In any case, the present invention does not require the library to be capable of "display" of the gene product at the bacterial surface, as with phage libraries; rather, the gene product is preferable expressed intracellularly, and is advantageously not expressed as a fusion with a vector gene product.

DNA binding domain libraries are preferably based on a known DNA binding domain architectures (e.g. basic leucine zipper, bZIP) and may be derived using PCR amplification with "family-specific" primers. Such libraries may be crossed with hybrid-promoters bearing defined target sequences or libraries of target sequences. In addition to providing information on the distribution of members of the family in a given genome, such libraries may be used to identify and study proteins or molecular compounds that modify DNA interaction within a family of DNA binding domains, for example Tax (from HTLV-1) in the case of bZIP proteins.

In an alternative embodiment, they may also be used to select DNA binding domains which conditionally bind their target sequence only in the presence of other factors such as protein cofactors or small molecular compounds, for example drugs that intercalate into DNA or alter the degree of supercoiling or recognise DNA sequences which have been modified chemically (e.g. methylated). The system can also be used "in reverse" i.e. to select proteins or molecular compounds that disrupt a particular DNA-protein interaction or to select DNA binding domains that do not bind a particular target sequence or library thereof.

More advanced libraries are preferably derived directly from genomic DNA. or cDNA libraries and selected on hybrid promoters bearing a repertoire of target sequences, comprising either a stretch of randomised sequence or a library of inserts derived from fragmented genomic DNA. Data obtained in this way allows the compilation of a genomic directory of DNA binding domains and the building of a promoter-DNA binding domain interaction map.

### D: Hybrid polypeptides

The generation of hybrid polypeptides by domain fusion is well known in the art and may be effected by fusing polypeptides or, preferably, by fusing nucleic acids which, encode the polypeptides. It has been known since 1976 that DNA binding and transcriptional activator domains are separable, and can be swapped between proteins: see Ma and Ptashne, who reported (Cell, (1987) 51, 113-119; Cell. (1988)55, 443-446) that when both the GAL4 N-terminal domain and C-terminal domain are fused together in the same protein, transcriptional activity is induced. Other proteins are also known function as transcriptional activators via the same mechanism. For example, the GCN4 protein of Saccharomyces cerevisiae as reported by Hope and Struhl. Cell, 46, 885-894 (1986), the ADR1 protein of Saccharomyces cerevisiae as reported by Thukral et al., Molecular and Cellular Biology, 9, 2360-2369, (1989) and the human estrogen receptor. as discussed by Kumar et al., Cell, 51, 941-951 (1987) both contain separable domains for DNA binding and for maximal transcriptional activation.

The same is specifically known of the σ54 bacterial transcriptional activators, although a genetic screen based thereon has not been proposed. Therefore. the present invention may be carried out using techniques which are known to those skilled in the art, particularly as applied to 2-hybrid techniques in eukaryotic cells.

Synthesis of chimeric genes for the purposes of the present invention may be carried out by any desired means, including polynucleotide synthesis and mutagenesis approaches. For example, a number of methods for site-directed mutagenesis are known in the art, from methods employing single-stranded phage such as M13 to PCR-based techniques (see "PCR Protocols: A guide to methods and applications", M.A. Innis, D.H. Gelfand, J.J. Sninsky, T.J. White (eds.). Academic Press, New York, 1990). Preferably, the commercially available Altered Site II Mutagenesis System (Promega) may be employed, according to the directions given by the manufacturer.

### E: Host Cells

Host cells useful in conjunction with the present invention are prokaryotic cells, advantageously bacterial cells. *E. coli* is the preferred host; however, host cells may belong to any species or genus in which σ54 RNAP-driven transcription is possible, such as *Klebsiella, Rhodobacter, Rhizobium, Acinetobacter, Pseudomonas, Escherichia*, *Bacillus, Caulobacter, Vibrio, Rhodospirillum, Alcaligenes, Salmonella, Myxococcus, Xylella, Azospirillum, Aquifex, Agrobacterium* and other organisms. In E.coli, the preferred configuration is a modified strain, in which a truncated form of Nif (or another activator) is coexpressed to boost specific activation (see Methods).

Preferably, the host cells lack repressors of the σ54 activator being used, such that the transcription activating domain is constitutively active. Repressors may be deleted by genetic mutation and/or selection, or inhibited by expression of antisense constructs, or the like. In general, due to the accessibility of bacterial genetics, especially in *E. coli*, deletion of repressor genes is straightforward to those skilled in the art.

### F: Reporter Genes

Reporter genes of various types are known in the art and may be used in conjunction with the present invention. A "reporter gene", as referred to herein, may be the coding sequence which encodes a detectable gene product, or the coding sequence including the necessary control sequences for its expression in accordance with the invention, as appropriate.

Advantageously, the reporter gene is selected from the group consisting of metabolic markers such as the lac operon (lacZ, lacY and lacA); proteins conferring a fluorescent phenotype, such as GFP; proteins conferring antibiotic resistance, such as Zeo.

Certain reporters, such as the LacZ gene, are widely used in bacterial genetics and are useful in the performance of the invention. However, other genes may also be employed, including fluorescent proteins. For example, green fluorescent proteins (GFPs) of cnidarians, which act as their energy-transfer acceptors in bioluminescence, can be used in the invention. A green fluorescent protein, as used herein, is a protein that fluoresces green light, and a blue fluorescent protein is a protein that fluoresces blue light. GFPs have been isolated from the Pacific Northwest jellyfish, *Aequorea victoria,* from the sea pansy, *Renilla reniformis*, and from *Phialidium gregarium*. (Ward et al., 1982, Photochem. Photobiol., 35: 803-808; Levine et al., 1982, Comp. Biochem. Physiol.,72B: 77-85).

A variety of *Aequorea*-related GFPs having useful excitation and emission spectra have been engineered by modifying the amino acid sequence of a naturally-occurring GFP from Aequorea victoria. (Prasher et al., 1992, Gene, 111: 229-233; Heim et al., 1994, Proc. Natl. Acad. Sci. U.S.A., 91: 12501-12504; PCT/US95/14692). As used herein, a fluorescent protein is an Aequorea-related fluorescent protein if any contiguous sequence of 150 amino acids of the fluorescent protein has at least 85% sequence identity with an amino acid sequence. either contiguous or non-contiguous, from the wild-type Aequorea green fluorescent protein (SwissProt Accession No. P42212). Similarly, the fluorescent protein may be related to *Renilla* or *Phialidium* wild-type fluorescent proteins using the same standards.

*Aequorea*-related fluorescent proteins include, for example, wild-type (native) *Aequorea victoria* GFP, whose nucleotide and deduced amino acid sequences are presented in GenBank Accession Nos. L29345, M62654, M62653 and others *Aequorea*-related engineered versions of Green Fluorescent Protein, of which some are listed above. Several of these, i.e., P4, P4-3, W7 and W2 fluoresce at a distinctly shorter wavelength than wild type.

A specific advantage of fluorescent proteins is that they facilitate FACS sorting of cells in a manner dependent on reporter gene expression (Norman, S.O. (1980). Flow cytometry. *Med Phys.* **7**, 609-615; Mackenzie, N.M. & Pinder, A.C. (1986). The application of flow microfluorimetry to biomedical research and diagnosis: a review. *Dev. Biol. Stand* **64,** 181-193).

Other reporter genes may complement auxotrophic mutations. confer antibiotic resistance or other selectable characteristics to the host bacteria. Reporter genes may be wholly or partly heterologous to the host cell, and introduced by mutagenesis and/or transformation with appropriate vectors. Alternatively, endogenous σ54-responsive genes may be used as reporter genes.

The reporter gene also contains a binding site for σ54 RNAP. The consensus sequence for σ54 RNAP binding is 5' T**GG**CAC-N5-TT**GC**a/t 3'. This sequence is located at -12 to -24 with respect to the start of transcription. whilst the more common sigma 70 recognition sequence is situated at -10 to/ -35. Both the GG & GC must be on the same face of the DNA helix.

In order to increase specificity, combinations of two or more reporter genes (preferably in tandem) may be used.

Where the reporter gene is chimeric. i.e. comprises heterologous binding sites for the nucleic acid binding sequence and σ54 RNAP binding sites incorporated into the same nucleic acid, the spacing between the σ54 RNAP binding site and the nucleic acid binding sequence binding site is preferably conserved with respect to the natural gene from which the σ54 RNAP binding site is taken. Advantageously, the spacing is at least calculated such that the spatial relationship of the elements on respective faces of the nucleic acid helix is maintained.

Reporter genes advantageously comprise a binding site for a further activation factor, such as IHF. These factors are believed to induce bending of the DNA, thus potentiating activation of σ54 RNAP-driven transcription by σ54 activators. Alternatively, the DNA itself may be intrinsically bent, thus providing constitutive potentiation of σ54-specific activation.

### G: Configurations of the Invention

The present invention may be configured in three basic ways: a first configuration, in which reporter gene activation is dependent on the interaction between the nucleic acid and a nucleic acid binding domain on the hybrid protein; a second configuration, in which reporter gene activation is dependent on interaction between bait and prey polypeptides which serves to bring together two or more components of the hybrid protein; and a third configuration, in which reporter gene activation by a σ54 activator is dependent on the presence of DNA-bending polypeptides. As referred to herein, an interaction is advantageously a binding interaction.

Where the invention is configured to detect protein-nucleic acid interaction, libraries of proteins and/or nucleic acids may be prepared as described above. Proteins having improved nucleic acid binding, or nucleic acid sequences having improved affinity for protein domains, may be developed by mutagenesis and selection of candidate sequences. Alternatively, protein and/or nucleic acid sequences may be used to identify, *in vivo,* cognate binding partners.

Chimeric σ54 activators also offer the opportunity to better understand aspects of the process of transcriptional activation at σ54 promoters. In the case of NifA, t is known that binding of the target sequence together with ATP binding promotes oligomerisation of NifA. It is believed that it is the oligomer which contacts the polymerase and catalyses the ATP-driven isomerisation of the polymerase holoenzyme. Taking advantage of the superactivation effect described above it may be possible to address questions such as which components of the oligomer (e.g. the DNA-bound NifA vs. NifAΔC, i.e. NifA with the DNA binding domain removed), which are contacting the polymerase and/or coupling ATP hydrolysis to transcriptional activation etc. Furthermore, usage of NifAΔC cofactors from different species (together with their diversification by PCR shuffling) allows identification of the sequence regions critical for transcriptional activation and a "maturation" of the NifAΔC coactivator. Indeed, we have found the NifA from *K. pneumoniae* to be a superior cofactor to *A. vinelandii* NifA. Finally, it may be possible to use chimera of a known DNA binding domains (e.g. GCN4) and a cDNA library as a prokaryotic "enhancer" trap, to isolate σ54 activators on a genome-wide scale.

Configuration of the invention to detect protein-protein interactions follows the general scheme of the yeast two-hybrid assay, and the reagents used in the invention may be set up accordingly. In general. therefore, the invention will comprise a nucleic acid binding domain-bait fusion, and a prey-σ54 activator domain fusion. Although, in general, "bait" refers to a known polypeptide and "prey" to an unknown polypeptide, the terms may be used interchangeably. Indeed, the invention comprises configurations in which both bait and prey are known, or both are unknown.

Binding between the bait and the prey result in constitution of a hybrid protein which comprises both a nucleic acid binding domain and a σ54 activator domain. The hybrid protein is able to activate transcription from a reporter gene, thus providing a bait:prey binding-dependent signal.

Protein-protein interactions may be selected using the preferred NifA system, in which the hybrid σ54 transcriptional activator includes the NifA activation domain. The NifA bacterial two-hybrid system may be used for the generation of interaction matrices between cDNA libraries. Ultimately such interaction matrices may yield an interaction map of the proteins of an organism. The invention provides an alternative to the yeast two hybrid system.

Systems based on σ54 have a number of advantages over the other systems that are available, e.g. the conceptually similar yeast one and two-hybrid system. A bacterial host allows substantially larger repertoires to be obtained and thus a much larger molecular diversity to be screened. In particular, using combinatorial infection, the system of the invention allows the "crossing" of both σ54-chimera repertoires with libraries of hybrid reporter constructs, thus permitting coevolution of DNA binding domains, and recognition sites, or coselection of DNA binding domains and target sites from genomic libraries.

Because selection in the σ54-based system is based on a positive readout, i.e. activation of transcription, it is less prone to false positives than other approaches relying on the inhibitory effect of the expressed DNA binding domains, like the transcription interference assay (Elledge S.J. et al (1989) *Proc. Nat. Acad. Sci USA* 86, 3689). In vivo selection in general may result in the selection of novel DNA binding domains that are more attuned to working under realistic conditions, including supercoiling of the recognition site, presence of a large excess of chromosomal DNA and high protein concentration. Another advantage of the system of the invention is that extremely low levels of the hybrid protein appear to be sufficient to affect maximum activation of transcription. This is particularly helpful in the case of DNA binding domains that are prone to aggregation.

The σ54-based systems of the present invention may be further adapted to take into account potential disadvantages of bacterial expression. For instance. E. coli expression may be suboptimal for large eukaryotic transcription factors. However, large eukaryotic proteins can often be split into smaller domains which retain function and are usually readily expressed in E. coli.

According to the third configuration, a constitutively active σ54 activator may be used to screen a library of candidate DNA-bending polypeptides, preferably in a HIF negative host. Since the degree of activation by the σ54 activator may be dependent on DNA bending by additional factors, the levels of expression of the reporter gene will be modulated by the DNA-bending activity of the candidate DNA-bending polypeptides.

The invention is further described, for the purposes of illustration only, in the following examples.

### Examples

NifA from *A. vinelandii* is a well-studied member of the family of bacterial enhancers and it is a positive regulator of the expression of nitrogenase components in diazotrophs. It is inhibited by NifL in response to the presence of oxygen or ammonia. When expressed in *E. coli,* which lacks endogenous NifL or an equivalent, NifA is constitutively active. Because of the highly conserved nature of the activation mechanism of σ54 RNA polymerase, NifA is a very strong activator of transcription in *E. coli.*

Like other members of the family of bacterial enhancer proteins. NifA is modular in architecture, both structurally and functionally, comprising 3 domains. a N-terminal sensor domain , a central activation domain (AD), and a C-terminal DNA binding domain (DBD). The central activation domain (AD) can activate transcription independent of DNA binding if overexpressed. Thus the DBDs function appears to be primarily to increase the Activator domain's concentration in the promoter proximity.

We have exploited the modularity of the enhancer structure and swapped the natural NifA DNA binding domain (DBD) for heterologous DBDs and libraries thereof. Here we describe the activity of these NifA-chimeras in the activation of transcription from the σ54 dependent promoter nifH and hybrids thereof.

### Materials & Methods

### Media & Reagents

2xTY, MacConkey agar are described elsewhere (Miller J.H. (1972) *Experiments in molecular genetics*, Cold Spring Harbour, NY). Antibiotics were used at the following concentrations: Ampicillin 0.1 mg/ml, Chloramphenicol 10µg/ml, Streptomycin 25µg/ml. Min-lac medium was essentially M9 medium (Sambrook *et al*., Molecular Cloning: A Laboratory Manual, 2d ed. (1989) Cold Spring Harbor Laboratory Press, Cold Spring Harbor, N.Y) supplemented with 1mM MgSO₄, 20µM CaCl₂, 2% (w/v) lactose, 2mg/ml casamino acids, 40µg/ml L-tryptophan, 5µg/ml thiamine and appropriate antibiotics. MinlacX plates where essentially M9 plates supplemented 2% lactose, appropriate antibiotics and 40µg/ml X-gal (5-bromo-4-chloro-3-indolyl-b-D-galactopyranoside).

### Strains

TG1ΔK was derived from TG1 (Gibson T. J. (1984) *Studies on the Epstein-Barr virus genome,* University of Cambridge) using the genome integration strategy of Haldimann A. *et al*., (1996) *Proc. Nat. Acad Sci USA* 93, 14361. Briefly, NifA (*K. pneumoniae*) residues 1-462 was amplified using Pfu polymerase (Stratagene) and primers 1 (5'- GAG TCA CTA ACG CAT ATG ATC CAT AAA TCC GAT TCG GAC -3'), 2 (5'- CGC GGA TCC AAG CGG CCG CTC ATT AGC GAT GGT TGA ACA GAA TCA C -3') cut with Ndel and BamHI and cloned into the genome targeting suicide vector pSK50D-uidA2 (Haldimann, *Op. Cit.*) and transformed into the Pir⁺ host strain BW23473 (Metcalf W.W. et al (1994) *Plasmid* 35, 1). Vectors were isolated and transformed into the Pir⁻ strain TG1 harbouring the plasmid pINT-ts (Hasan N. et al (1994) *Gene* 150, 51). Chromosomal integration was induced by a temperature shift to 42°C, which leads to expression of λ integrase from pINT-ts and simultaneously stops its replication. Integrants where identified by Kanamycin resistance and screened for Nif coactivation. Once obtained TG1ΔK was grown routinely without antibiotic selection.

### Constructs

Chimeric constructs were based on pDB737 (Austin S. et al (1994) *J. Bacteriol*. 176, 3460 Buck M. et al (1986) *Nature* 320, 374) encoding NifA (*A. vinelandii*) under the control of the T7 promoter in the plasmid pT7-7 (Tabor S. & Richardson C.C. (1985) *Proc Natl Acad Sci USA* 82, 1074). Expression was by leakiness of the T7 promoter. Chimeras were constructed taking advantage of an unique BanII cutting site, in the linker region between the central domain of NifA and the DBD. GCN4 was amplified using Pfu polymerase (Stratagene) and primers 3 (5'- GCT GCC AGC GAG AGC CCG CCG CTC GCC GCG ATT GTG CCC GAA TCC AGT GAT CCT -3') and 4 (5'- GAG CTA AAG CTT TTA TTA GCG TTC GCC AAC TAA TTT CTT TAA TCT GGC -3') cut with BanII and Hind3 and ligated into pDB737 cut with BanII and Hind3. ERDBD was amplified using primers 5 (5'- GTC GAC AAC GAG AGC CCG CCG CTC GCC GCG GAA ACG CGT TAC TGC GCT GTT -3') TGC and 6 (5'- GGT CAG CGC GTG GAT CCT TAA CCA CCA CGA CGG TCT TTA CG-3') cut with BanII and BamHI and ligated into pDB737 cut with BanII and BamHI. The vector p737S1 is derived from pDB737 by replacing the bla gene with aadA conferring streptomycin resistance and the insertion of a f1 phage origin for packaging of the vector into filamentous phage particles. Briefly, aadA was, amplified using primers 7 (5'- TCA GCG CAC GCT GAC GTC GTG GAA ACG GAT GAA GGC ACG AAC -3'), 8 (5'-CCG CCT GGA GGT GGC CAT TAT TTG CCG ACT ACC TTG GTG ATC TCG CC -3') and cut with AatII and MscI and ligated with pDB737 cut with AatII and ScaI. The resulting vector p737S was cut with AatII, ClaI. The f1 ori was amplified using primers 9 (5'- GCT GCC GAC TCG ATC GAT GAA TGG CGA ATG GCG CCT GAT GCG G -3'), 10 (5'-CCG GGT CGT GAC GTC AGT GTT GGC GGG TGT CGG GGC TGG C -3') cut with AatII, ClaI and cloned into the cut p737S to give p737S1. NifA-X chimera were transferred from pDB737 to p737S1 by digestion with NdeI. Hind3 (BamHI for NifA-ERDBD).

Reporter constructs were derived from pACYC184 and the vector pMB1 (Buck M. *et al*. (1986) *Nature* 320, 374). Briefly the lac-operon (lacZYA) was amplified with primers 11 (5'- GAG TCA ATT CGG GGA TCC CGT CGT TTT ACA ACG TCG TGA CTG G-3'). 12 (5'- GAG TCA TTC TGG CCA GTC GAC CGC TCT GCC GGT GGT TAC -3') and cut with BamHI and MscI. The nifH promoter segment from pMB1 was amplified with primers 13 (5'- GAG TCA TTC AAG CTT GCG TGG AAT AAG ACA CAG GGG GCG-3'), 14 (5'- GAG TCA TTC GGG ATC CCC GGA TTT ACC GAT ACC GCC TTT ACC -3') and cut with Hind3, BamHI and the 2 fragments simultaneously ligated with pACYC184 cut with Hind3 and BsaAI to give pMB3. The f1 ori was amplified with primers 15 (5'- GCT GCC GAC TCG GCT AGC GAA TGG CGA ATG GCG CCT GAT GCG G -3'), 16 (GCC GGG TCG CTT TAA AGT GTT GGC GGG TGT CGG GGC TGG C -3') and cut with NheI and DraI and ligated into pMB3 cut with both NheI, XmnI to give pMB31.

### Selection and screening

Cells were cotransformed either by simultaneous or sequential electroporation with an expressor construct and a reporter construct and grown overnight with appropriate antibiotic selection at 34°C in M9-lac medium and plated out. β-gal expression was scored either on MacConkey or Minlac-X-gal indicator plates or by ONPG enzyme assay of selected colonies (see below).

### Enzyme assay

ONPG assays used to measure β-gal activity were essentially as described by Kolmar H. et al. (1995) *EMBO J* 14, 3895. Briefly, 20µl of an overnight culture is transferred to a microtitre well and 100µl of chloroform saturated Z-buffer (100mM NaHPO₄, 1mM KCL, 1mM MgSO₄, 50mM β-mercaptoethanol, pH 7.0 (Miller J.H. (1972) *Experiments in molecular genetics*, Cold Spring Harbour, NY) was added and the optical density at 600nm determined using an ELISA reader. Cells were lysed by addition of 50µl Z-buffer with 0.4% (w/v) SDS and incubated at 30°C for 10 min. 50µl of Z-buffer with 4mg/ml O-nitrophenyl-β-D-galactopyranoside were added and the optical density at 420nM was recorded automatically every 15s over a period of 60min. Specific β-gal activity was calculated from the Vₘₐₓ as in Miller (*Op. Cit*.).

### Example 1: NifA chimera with heterologous DNA binding domains activate transcription but only from promoters with a cognate recognition site

To investigate in what way transcription activation by NifA was dependent on the NifA DNA binding domain (DBD) and on native nif promoter structure, we prepared NifA-chimeras in which NifA DNA binding domain (DBD) had been replaced by heterologous DBDs of diverse structural architectures. Initially we explored DBDs which, like the NifA wild type (wt) DBD bind to symmetrical DNA recognition sequences such as the basic leucine zipper (bZIP) DBD of the yeast transcription factor GCN4, the Zn-finger domain of the human estrogen receptor DNA binding domain (ERDBD) and determined their capacity to activate transcription of a lacZ reporter gene in vivo from a hybrid nifH promoter, in which the NifA UAS had been deleted and replaced by recognition sites for the heterologous DBDs.

In order to simplify comparison of transcription activation by NifA chimeras with activation by wt NifA, all reporter constructs had a single DNA recognition site. The wt nifH promoter UAS contains three bona fide NifA recognition sites. Deletion of the two sites more distal to the promoter, however, did not appear to reduce transcription activation in our reporter under conditions tested.

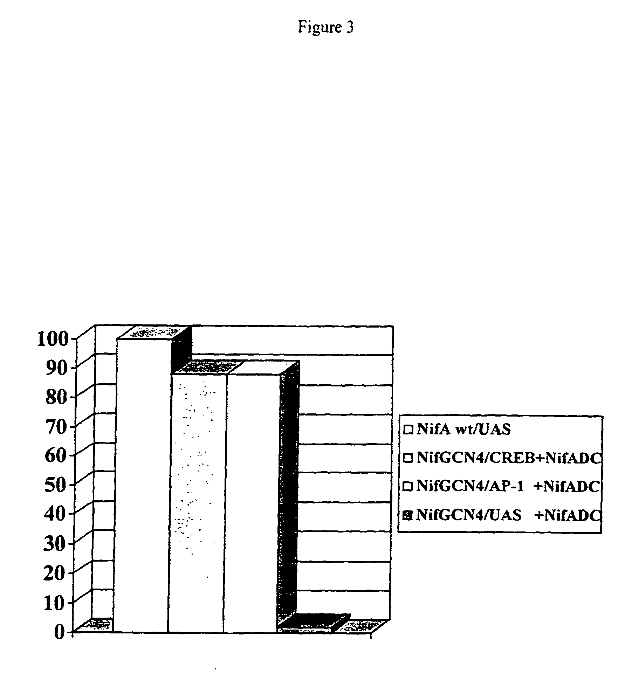

Transcription activation by NifA-chimeras was specific in that they only activated lacZ expression from hybrid-promoters bearing their cognate recognition sequences but not from control reporter constructs bearing wild type UAS or a non-cognate site (Fig. 3). In analogy to wt NifA the presence of two or more recognition sites (in phase, see below) did not increase activation by the Nif-GCN4 chimera.

Activity was also dependent on the phasing of the recognition site with respect to the promoter: when the symmetric ATF/CREB recognition site for GCN4 was offset in increments of 1 bp, optimal activity was observed when the ATF/CREB was centred on the same bp as the symmetric wt UAS. Presumably efficient contact with the RNA Pol holoenzyme requires that the activator be bound on the right face of the DNA.

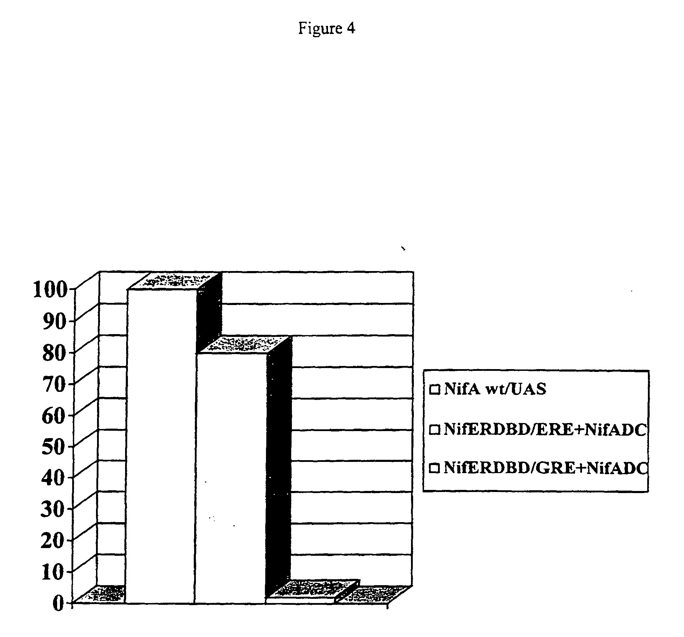

Transcription activation by NifA-chimeras appears to preserve fine specificity of isolated DBDs. Wild-type GCN4 binds with equal affinity to the symmetric ATF/CREB site as well as to the pseudo-symmetric AP-1 site. Indeed, the NifA-GCN4 chimera showed identical levels of transcription activation in reporter constructs with either of these sites (Fig. 3). A NifA-ERDBD chimera showed strong activity on a reporter with its cognate ERE site but no activity above background levels with reporters bearing the similar GRE recognition site for the closely related glucocorticoid receptor DBD (Fig. 4).

### Example 2: Coexpression of wild-type NifA with NifA-chimeras boosts specific transcription activation by NifA chimeras in a specific and DNA independent manner

The level of transcription activation by the NifA-GCN4 and NifA-ERDBD chimeras was lower (ca. 10%) than for wt NifA. However, near wt levels of activity (up to 80%) were reached when wt NifA was coexpressed within the same cell as a "coactivator".

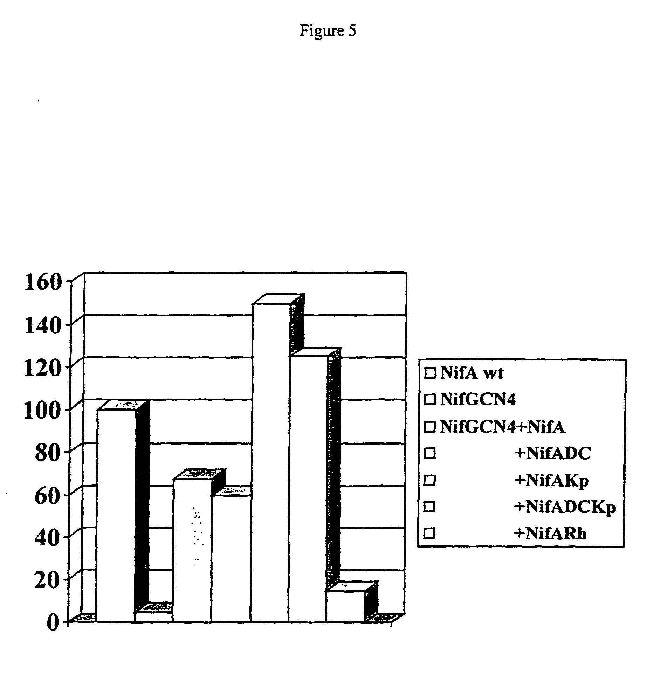

The coactivation was independent of DNA binding, as NifA variants in which the DBD had been deleted (NifAΔC) was found to be just as active as wt NifA. On the other coexpression of an isolated NifA central domain (both the DBD as well as the N-terminal sensor domain deleted (NifAΔNC)) failed to coactivate. NifA derived from different species showed greatly variable efficiencies as coactivators. NifA variants from *K. pneumoniae* (NifA Kp, NifAΔC Kp) were almost three times as effective as NifA, while NifA variants from Rhizobium (NifA Rh1, NifA Rh2) were poorly active as coactivators (Fig. 5).

The coactivator effect was found to enhance only specific transcriptional activation and not background levels of transcription from promoters with non-cognate recognition sites. We therefore constructed an *E. coli* strain, expressing NifAΔC Kp (the *K. pneumoniae* NifA with its DBD deleted) from a weak promoter (phoB) from the chromosome (TG1:ΔK).

The coactivation effect has analogies in eukaryotic transcription, for example the enhancer Sp1, in which isolated Sp1 activation domains can stimulate transcriptional activation by the DNA binding-form of Sp1, a phenomenon termed "superactivation".

### Example 3: Tethering of NifA chimera at the UAS is sufficient for activation, but strong activation requires correct positioning

We also investigated transcription activation by NifA-chimeras with asymmetrical recognition sites such as the classic Zn-finger Zif268 as well as the DBD from p53.

Both NifA-Zif268 and NifA-p53 chimeras activated transcription but only at low levels (2 - 5-fold above the background). However, when the Zif recognition site was duplicated, to give a symmetric palindromic site transcription activation increased substantially. Non-palindromic duplication of the recognition site in tandem did not increase activation.

Thus while simple tethering is sufficient for some activation, only bipartite binding appears to give a strong activation. Presumably, tethering only leads to an approximate positioning of the activation domain with respect to the RNA polymerase holoenzyme, thereby reducing the likelihood of a productive interaction.

### Example 4: Selection of active NifA-chimeras by lac complementation

Using expression of the lac operon (lacZYA) from our reporter construct as the read-out of transcription activation allows the selection of active NifA-chimera on the basis of metabolic complementation of a Δlac strain, with lactose as the only carbon source. Initially we spiked populations of NifA-ERDBD with NifA-GCN4 at the ratios 1/10⁴, 1/10⁶ in the presence of the GCN4 cognate reporter ATF/CREB-nifH and grew populations overnight in minimal medium supplied with lactose. Pre- and post selection populations were scored by plating on MacConkey-lactose plates as well as by PCR screening. The results are summarised in Table 1. Selection factors of up to 10,000 -fold per round were observed.

**Table 1:**

| Selection factors for Nif selection by lac complementation | |
|---|---|
| NifGCN4/NifERDBD | Selection factor |
| 1/10⁴ | 40 fold |
| 1/10⁵ | 40 fold |
| 1/10⁶ | 200 fold |
| 1/10⁷ | 4000 fold |

### Example 5: Selection of active NifA-chimeras by flow cytometry.

Expression of β-galactosidase (lacZ) as the read-out of transcription activation allows the selection of active NifA-chimera on the basis of metabolic complementation of a Δlac strain, grown on lactose as the only carbon source. However, metabolic selection predisposes the system to the generation of false positives. Presumably, the prolonged growth under metabolic selection selects for mutant promoters, active in the absence of a cognate enhancer.

We have observed that that this only occurs for library sizes exceeding 10⁶. Indeed, others have found (using a related bacterial two-hybrid system) that it is not possible to retrieve positive clones from dilutions higher than 1/10⁶ by metabolic lac selection (G. Karimova, et al., (1998) Proc Natl Acad Sci USA 95, 12532-7). As it is well known that bacteria can develop a mutator phenotype under adaptive stress (P. D. Sniegowski, P. J. Gerrish, R. E. Lenski, (1997) *Nature* 387, 703-5), we conclude that it is preferable to separate the selection from the amplification (growth) step in order to reduce the likelihood of revertants.

We thus replaced lacZ with the *Aequorea victoria* green fluorescent protein (EGFP, F64L, S65T, ex488 nm, em527nm (the Clontech variant pGFPmut3.1. S65E , S72A, ex501nm, em511nm FACS optimised variant was also tried, but found inferior) as the reporter gene. GFP has the advantage that cells can be grown first and then separated on the basis of fluorescence using fluorescence activated cell sorting (FACS).

We prepared a trial library of mutant GCN4 bZIP DBDs (library size10⁸) in which 5 key residues (Asn235, Ala238, Ala239, Ser242, Arg243) of GCN4 interacting with DNA were randomised and selected it against a GFP hybrid reporter with the cognate ATF/CREB site. Library populations were grown overnight at 34°C in non-fluorescent medium NFM (minimal medium supplied with 2%glucose, 0.2% casaminoacids,12 ng/ml L-Trp). For FACS (Cytomation Mofo, 488 nm Laser, FL-1 530/40 filter) an 1 ml aliquot was diluted 10X in NFM and the top 1% fluorescent cell population was sorted into a 96 well plate at 1 cell per well, and grown up overnight at 34°C. Cell fluorescence of the grown up clones was measured by using a SPEGTRAmax^{R}GEMINI Dual-Scanning Microplate Spectrofluorometer (Molecular Devices), ex480, em520, (cut-off 515 nm). Plasmids from fluorescent wells were sequenced afterwards. Pre- and post selection populations were also scored by PCR screening as well as by plating on min glu (M9 Minimal medium + glucose) plates and visualised using fluorescence microscope.

10⁵ cells were sorted in total, from which 219 cells were in the top 1% fluorescent population and 132 of which were captured to the 96-well plates. 13 cells from these were fluorescent. Selected positives were checked by separating the mutant GCN4-bZIP DBD expressor plasmids. and re-transformine them together with cognate and non-cognate reporter plasmids. None of the selected positives gave a fluorescent signal when combined non-cognate reporter plasmids, but all were fluorescent when combined with the ATF/Creb cognate reporter plasmid (which did not produce any fluorescence when transformed on its own).

This indicates that GFP selection indeed avoids the isolation of false positives. Furthermore, when the library was checked prior to FACS sorting no fluorescent clones were identified when plating >10⁷ cells. 1/10 clones plated post selection were fluorescent, suggesting a selection factor in a single round in excess of 10⁶-fold.

## Claims

1. A method for detecting a protein-nucleic acid interaction between a nucleic acid molecule and a protein molecule in vitro or in vivo in bacteria, comprising the steps of:
a) providing one or more hybrid σ54 activator proteins comprising a heterologous nucleic acid binding domain and a constitutively active σ54 transcription activating domain;
b) providing one or more nucleic acid molecules comprising a binding site for the nucleic acid binding domain and a binding site for σ54 RNA polymerase (RNAP), which directs the expression of a reporter gene and leads to upregulation thereof in response to activation by the σ54 transcription activating domain; and
c) detecting expression of the reporter gene.

2. A method according to claim 1, comprising providing a repertoire of hybrid σ54 activator proteins, said repertoire comprising a plurality of different nucleic acid binding domain.

3. A method according to claim 1, comprising providing a repertoire of hybrid nucleic acid molecules, said repertoire comprising a plurality of different binding sites for the nucleic acid binding domain.

4. A method according to claim 1, comprising providing both a repertoire according to claim 2 and a repertoire according to claim 3.

5. A method for detecting a protein-protein interaction in vitro or in vivo in bacteria, comprising the steps of:
a) providing a first hybrid protein comprising a nucleic acid binding domain and a first polypeptide sequence bait;
b) providing a second hybrid protein comprising a prey polypeptide sequence and constitutively active σ54 transcription activating domain;
c) providing a nucleic acid molecule comprising a binding site for the nucleic acid binding domain and binding site for σ54 RNAP which directs the expression of a reporter gene and leads to upregulation thereof in response to activation by the σ54 transcription activating domain;
d) incubating the first and second hybrid proteins together with the nucleic acid molecule such that the prey and bait polypeptide sequences may bind; thereby forming a hybrid protein comprising both a nucleic acid binding domain and a σ54 transcription activating domain; and
e) detecting expression of the reporter gene.

6. A method according to claim 5, comprising providing a repertoire of first hybrid proteins, said repertoire comprising a plurality of bait polypeptides.

7. A method according to claim 5, comprising providing a repertoire of second hybrid proteins, said repertoire comprising a plurality of prey polypeptides.

8. A method according to claim 5, comprising providing a repertoire of first hybrid proteins and a repertoire of second hybrid proteins, said repertoires comprising a plurality of bait and prey polypeptides.

9. A method for screening a repertoire of candidate DNA-bending polypeptides in vivo in bacteria; comprising the steps of:
a) providing a repertoire of candidate polypeptide factors with potential to induce bending of DNA;
b) providing a σ54 activator protein comprising a nucleic acid binding domain and a σ54 transcription activating domain;
c) providing a nucleic acid molecule comprising a binding site for the nucleic acid binding domain and binding site for σ54 RNAP which directs the expression of a reporter gene and leads to upregulation thereof in response to activation by the σ54 transcription activating domain;
d) incubating the repertoire and σ54 activator together with the nucleic acid molecule in a integration host factor (IHF) host cell, such that σ54 activator and the nucleic acid molecule may interact, and transcription activated from the σ54 RNAP binding site in a manner dependent on DNA bending by the polypeptide factors; and
e) detecting expression of the reporter gene.

10. A method according to claims 1 to 8, wherein the polypeptides are obtainable by expression within a bacterial host cell.

11. A method according to claim 10, wherein the polypeptides are encoded in one or more libraries of nucleic acid vectors.

12. A method according to claim 11, wherein a first library of nucleic acid.vectors encodes a first chimeric gene, said gene comprising a nucleic acid sequence that encodes a nucleic acid-binding domain and a nucleic acid sequence encoding a first (bait) test protein or protein fragment in such a manner that the first test protein is expressed as part of a hybrid protein with the nucleic acid-binding domain.

13. A method according to claim 11, wherein a second library of nucleic acid vectors encodes a second chimeric gene; said gene comprising a nucleic acid sequence that encodes a σ54 transcriptional activation domain and a second (prey) test protein or protein fragment into the vector, in such a manner that the second test protein is capable of being expressed as part of a hybrid protein with the transcriptional activation domain.

14. A method according to any preceding claim, wherein the o54 transcriptional activator is selected from the group consisting of:
dbj|BAA16379.1| (D90877) FORMATE HYDROGENLYASE TRANSCRIPTIONAL ACTIVATOR;
emb|CAA26472.1| (X02616) pot. Nifa gene product (aa 1-484) [Klebsiella pneumoniae];
emb|CAA53584.1| (X75972) anfa [Rhodobacter capsulatus];
emb|CAA92413.1| (Z68203) nifa homologue [Rhizobium sp.];
emb|CAA93242.1| (Z69251) mopr [Acinetobacter calcoaceticus];
emb|CAB53157.1| (X07567) nifa1 [Rhodobacter capsulatus];
emb|CAB56537.1| (AJ249642) response regulator [Pseudomonas stutzeri];
gb|AAA58220.1| (U18997) ORF_o532 [Escherichia coli];
gb|AAA99303.1| (L43064) regulatory protein [Pseudomonas aeruginosa];
gb|AAB91397.1| (AF033203) nifaii protein [Rhodobacter capsulatus];
gb|AAC05586.1| (AF006075) regulatory protein [Bacillus subtilis];
gb|AAC37124.1| (L81176) fleq [Pseudomonas aeruginosa];
gb|AAC45640.1| (AF010585) putative sigma 54 activator [Caulobacter crescentus];
gb|AAC46367.1| (AF014113) two-component response regulator [Vibrio cholerae];
gb|AAD34591.1|AF145956_1 (AF145956) transcriptional activator nifa [Rhodospirillum rubrum];
gb|AAD38416.1| (AF155934) nifa [Alcaligenes faecalis];
gb|AAF28395.1| (AF069392) flam [Vibrio parahaemolyticus];
gb|AAF33506.1| (AF170176) Salmonella typhimurium transcriptional regulatory protein;
gb|AAF61932.1| (AF230804) sigma-54 activator protein ActI [Myxococcus xanthus];
gb|AAF85342.1|AE004061_7 (AE004061) two-component system, regulatory protein [Xylella fastidiosa];
gb|AAF94676.1| (AE004230) sigma-54 dependent response regulator [Vibrio cholerae];
gb|AAF95280.1| (AE004286) sigma-54 dependent response regulator [Vibrio cholerae];
gb|AAF96095.1| (AE004358) sigma-54 dependent transcriptional regulator [Vibrio cholerae];
gb|AAG01527.1|AF288483_1 (AF288483) nifa [Azospirillum brasilense];
pir∥A48291 ornithine decarboxylase inhibitor - Escherichia coli;
pir∥B49940 nitrogen regulator I homolog - Escherichia coli;
pir∥C70320 transcription regulator nifa family - Aquifex aeolicus;
pir∥C70396 transcription regulator ntrc family - Aquifex aeolicus;
pir∥C70454 transcription regulator ntrc family - Aquifex aeolicus;
pir∥D70315 transcription regulator ntrc family - Aquifex aeolicus;
pir∥H69581 transcription activator of acetoin dehydrogenase operon acor - Bacillus subtilis;
pir∥139719 nitrogen regulatory protein - Agrobacterium tumefaciens;
pir∥JC5471 regulatory protein nifa - Azospirillum lipoferum;
pir∥T08624 probable ntrc-type response regulator - Eubacterium acidaminophilum;
sp|P03027|NIFA_KLEPN NIF-SPECIFIC REGULATORY PROTEIN;
sp|P09570|NIFA_AZOVI NIF-SPECIFIC REGULATORY PROTEIN;
sp|P12627|VNFA_AZOVI NITROGEN FIXATION PROTEIN VNFA;
sp|P14375|HYDG_ECOLI TRANSCRIPTIONAL REGULATORY PROTEIN HYDG;
sp|P21712|YFHA_ECOLI HYPOTHETICAL 49.1 KD PROTEIN IN GLNB-PURL INTERGENIC REGION (ORFXB);
sp|P24426|NIFA_RHILT NIF-SPECIFIC REGULATORY PROTEIN;
sp|P25852|HYDG_SALTY TRANSCRIPTIONAL REGULATORY PROTEIN HYDG;
sp|P27713|NIFA_HERSE NIF-SPECIFIC REGULATORY PROTEIN;
sp|P30667|NIFA_AZOBR NIF-SPECIFIC REGULATORY PROTEIN;
sp|P38035|RTCR_ECOLI TRANSCRIPTIONAL REGULATORY PROTEIN RTCR;
sp|P54929|NIFA_AZOLI NIF-SPECIFIC REGULATORY PROTEIN;
sp|P56266|NIFA_KLEOX NIF-SPECIFIC REGULATORY PROTEIN;
sp|Q06065|ATOC_ECOLI ACETOACETATE METABOLISM REGULATORY PROTEIN ATOC (ORNITHINE/ARGININE;
sp|Q46802|YGEV_ECOLI HYPOTHETICAL SIGMA-54-DEPENDENT TRANSCRIPTIONAL REGULATOR IN;
sp|QS3206|NIFA_RHISN NIF-SPECIFIC REGULATORY PROTEIN; and
sp|Q9ZIB7|TYRR_ER WHE TRANSCRIPTIONAL REGULATORY PROTEIN TYRR.

15. A method according to any one of claims I to 14, wherein the σ54 transcriptional activator is the NifA transcriptional activator or the PspF transcriptional activator.

16. A method according to any one of claims 1 to 14, wherein the hybrid σ54 transcriptional activator is NifA and activation resulting from NifA-oS4 RNAP interaction is enhanced by the coexpression of wild-type or mutant NifA.

17. A method according to claim 16, wherein the hybrid σ54 transcriptional activator is NifA from *Azotobacter vinelandii*, and the wild-type or mutant NifA is NifA from *Klebsiella pneumoniae*.

18. A method according to any preceding claim, wherein the nucleic acid molecule comprises a binding site for a factor which induces DNA bending.

19. A method according to claim 18, wherein the factor is integration host factor (IHF).

20. A method according to any one of claims 1 to 17, wherein the nucleic acid molecule comprises DNA that is intrinsically bent.

21. A method according to any preceding claim, wherein the nucleic acid molecule comprises a NifH promoter from *A.vinelandii* driving a reporter gene.

22. A method according to any preceding claim, wherein the reporter gene is selected from the group consisting of a metabolic markers such as the lac operon (lacZ, lacY and lacA); proteins conferring a fluorescent phenotype, such as GFP; proteins conferring antibiotic resistance, such as Zeo.

23. A method according to any preceding claim, which is carried out *in vivo* in bacteria.

24. A method according to claim 23, wherein the *in vivo* host is *E.coli*.

25. A method according to any one of claims 1 to 24, which is carried out *in vitro.*

## Patentansprüche

1. Verfahren zum Feststellen einer Protein-Nukleinsäure-Wechselwirkung zwischen einem Nukleinsäuremolekül und einem Proteinmolekül in vitro oder in vivo in Bakterien mit den Stufen, in denen man:
a) ein oder mehrere Hybrid-σ54-Aktivatorproteine bereitstellt, welche eine heterologe Nukleinsäurebindungsdomäne und eine konstitutiv aktive σ54-Transkriptionsaktivierungsdomäne umfassen,
b) ein oder mehrere Nukleinsäuremoleküle bereitstellt, welche eine Bindungsstelle für die Nukleinsäurebindungsdomäne und eine Bindungsstelle für σ54-RNA-Polymerase (RNAP), welche die Expression eines Reportergens steuert und als Reaktion auf eine Aktivierung durch die σ54-Transkriptionsaktivierungsdomäne zu einer Hochregulierung davon führt, umfassen und
c) Expression des Reportergens feststellt.

2. Verfahren nach Anspruch 1, welches das Bereitstellen eines Repertoires an Hybrid-σ54-Aktivatorproteinen umfaßt, wobei das Repertoire eine Vielzahl von verschiedenen Nukleinsäurebindungsdomänen umfaßt.

3. Verfahren nach Anspruch 1, welches das Bereitstellen eines Repertoires an Hybrid-Nukleinsäuremolekülen umfaßt, wobei das Repertoire eine Vielzahl von verschiedenen Bindungsstellen für die Nukleinsäurebindungsdomäne umfaßt.

4. Verfahren nach Anspruch 1, welches das Bereitstellen sowohl eines Repertoires nach Anspruch 2 als auch eines Repertoires nach Anspruch 3 umfaßt.

5. Verfahren zum Feststellen einer Protein-Protein-Wechselwirkung in vitro oder in vivo in Bakterien mit den Stufen, in denen man:
a) ein erstes Hybridprotein bereitstellt, welches eine Nukleinsäurebindungsdomäne und einen ersten Polypeptidsequenzköder umfaßt,
b) ein zweites Hybridprotein bereitstellt, welches eine Beutepolypeptidsequenz und eine konstitutiv aktive σ54-Transkriptionsaktivierungsdomäne umfaßt,
c) ein Nukleinsäuremolekül bereitstellt, welches eine Bindungsstelle für die Nukleinsäurebindungsdomäne und eine Bindungsstelle für σ54-RNAP, welches die Expression eines Reportergens steuert und als Reaktion auf eine Aktivierung durch die σ54-Transkriptionsaktivierungsdomäne zu einer Hochregulierung davon führt, umfaßt,
d) die ersten und zweiten Hybridproteine zusammen mit dem Nukleinsäuremolekül inkubiert, so daß die Beute- und Köderpolypeptidsequenzen binden können, wobei ein Hybridprotein ausgebildet wird, welches sowohl eine Nukleinsäurebindungsdomäne als auch eine σ54-Transkriptionsaktivierungsdomäne umfaßt, und
e) Expression des Reportergens feststellt.

6. Verfahren nach Anspruch 5, welches Bereitstellen eines Repertoires von ersten Hybridproteinen umfaßt, wobei das Repertoire eine Vielzahl von Köderpolypeptiden umfaßt.

7. Verfahren nach Anspruch 5, welches Bereitstellen eines Repertoires von zweiten Hybridproteinen umfaßt, wobei das Repertoire eine Vielzahl von Beutepolypeptiden umfaßt.

8. Verfahren nach Anspruch 5, welches Bereitstellen eines Repertoires von ersten Hybridproteinen und eines Repertoires von zweiten Hybridproteinen umfaßt, wobei die Repertoires eine Vielzahl von Köder- und Beutepolypeptiden umfassen.

9. Verfahren zur Durchmusterung eines Repertoires von Kandidaten für DNA-krümmende Polypeptide in vivo in Bakterien mit den Stufen, in denen man:
a) ein Repertoire von Kandidatenpolypeptidfaktoren mit dem Potential, ein Krümmen von DNA zu induzieren, bereitstellt,
b) ein σ54-Aktivatorprotein bereitstellt, welches eine Nukleinsäurebindungsdomäne und eine σ54-Transkriptionsaktivierungsdomäne umfaßt,
c) ein Nukleinsäuremolekül bereitstellt, welches eine Bindungsstelle für die Nukleinsäurebindungsdomäne und eine Bindungsstelle für σ54-RNAP, welches die Expression eines Reportergens steuert und als Reaktion auf eine Aktivierung durch die σ54-Transkriptionsaktivierungsdomäne zu einer Hochregulierung davon führt, umfaßt,
d) das Repertoire und den σ54-Aktivator zusammen mit dem Nukleinsäuremolekül in einer Integration Host Factor- (IHF-) Wirtszelle inkubiert, so daß σ54-Aktivator und das Nukleinsäuremolekül miteinander wechselwirken können, und Transkription, die von der σ54-RNAP-Bindungsstelle in einer Art und Weise aktiviert wird, die von DNA-Krümmung durch die Polypeptidfaktoren abhängig ist, durchgeführt wird, und
e) Expression des Reportergens feststellt.

10. Verfahren nach einem der Ansprüche 1 bis 8, wobei die Polypeptide durch Expression in einer Bakterienwirtszelle erhältlich sind.

11. Verfahren nach Anspruch 10, wobei die Polypeptide in einer oder mehreren Bibliotheken von Nukleinsäurevektoren codiert werden.

12. Verfahren nach Anspruch 11, wobei eine erste Bibliothek von Nukleinsäurevektoren ein erstes chimäres Gen codiert, wobei das Gen eine Nukleinsäuresequenz umfaßt, die eine Nukleinsäurebindungsdomäne codiert, und eine Nukleinsäuresequenz, die ein erstes (Köder-) Testprotein oder -proteinfragment in solch einer Weise codiert, daß das erste Testprotein als ein Teil eines Hybridproteins mit der Nukleinsäurebindungsdomäne exprimiert wird.

13. Verfahren nach Anspruch 11, wobei eine zweite Bibliothek von Nukleinsäurevektoren ein zweites chimäres Gen codiert, wobei das Gen eine Nukleinsäuresequenz umfaßt, die eine σ54-Transkriptionsaktivierungsdomäne und ein zweites (Beute-) Testprotein oder -proteinfragment in dem Vektor in solch einer Weise codiert, daß das zweite Testprotein in der Lage ist, als ein Teil eines Hybridproteins mit der Transkriptionsaktivierungsdomäne exprimiert zu werden.

14. Verfahren nach einem der vorangegangenen Ansprüche, wobei der σ54-Transkriptionsaktivator aus der Gruppe ausgewählt ist, bestehend aus:
dbj|BAA16379.1|(D90877) FORMIATHYDROGENLYASE-TRANSKRIPTIONSAKTIVATOR;
emb|CAA26472.1|(X02616) pot. Nifa-Genprodukt (AS 1-484) [Klebsiella pneumoniae];
emb|CAA53584.1|(X75972) anfa [Rhodobacter capsulatus];
emb|CAA92413.1|(Z68203) nifa-Homologes [Rhizobium sp.];
emb|CAA93242.1|(Z69251) mopr [Acinetobacter calcoaceticus];
emb|CAB53157.1|(X07567) nifa1 [Rhodobacter capsulatus];
emb|CAB56537.1|(AJ249642) Antwortregulator [Pseudomonas stutzeri];
gb|AAA58220.1|(U18997) ORF_o532 [Escherichia coli];
gb|AAA99303.1|(L43064) Regulatorprotein [Pseudomonas aeruginosa];
gb|AAB91397.1|(AF033203) nifaii-Protein [Rhodobacter caspulatus];
gb|AAC05586.1|(AF006075) Regulatorprotein [Bacillus subtilis];
gb|AAC37124.1|(L81176) fleq [Pseudomonas aeruginosa];
gb|AAC45640.1|(AF010585) mutmaßlicher Sigma 54-Aktivator [Caulobacter crescentus];
gb|AAC46367.1|(AF014113) Zwei-Komponenten-Antwortregulator [Vibrio cholerae];
gb|AAD34591.1|AF145956_1 (AF145956) Transkriptionsaktivator nifa [Rhodospirillum rubrum];
gb|AAD38416.1|(AF155934) nifa [Alcaligenes faecalis];
gb|AAF28395.1|(AF069392) flam [Vibrio parahaemolyticus];
gb|AAF33506.1|(AF170176) Salmonella typhimurium Transkriptionsregulatorprotein;
gb|AAF61932.1|(AF230804) Sigma-54-Aktivatorprotein Actl [Myxococcus xanthus];
gb|AAF85342.1|AE004061_7 (AE004061) Zwei-Komponenten-System, Regulatorprotein [Xylella fastidiosa];
gb|AAF94676.1|(AE004230) von Sigma-54 abhängiger Antwortregulator [Vibrio cholerae];
gb|AAF95280.1|(AE004286) von Sigma-54 abhängiger Antwortregulator [Vibrio cholerae];
gb[AAF96095.1|(AE004358) von Sigma-54 abhängiger Transkriptionsregulator [Vibrio cholerae];
gb|AAG01527.1|AF288483_1 (AF288483) nifa [Azospirillum brasilense];
pir∥A48291 Ornithindecarboxylaseinhibitor - Escherichia coli;
pir∥B49940 Stickstoffregulator I-Homologes - Escherichia coli;
pir∥C70320 Transkriptionsregulator nifa-Familie - Aquifex aeolicus;
pir∥C70396 Transkriptionsregulator ntrc-Familie - Aquifex aeolicus;
pir∥C70454 Transkriptionsregulator ntrc-Familie - Aquifex aeolicus;
pir∥D70315 Transkriptionsregulator ntrc-Familie - Aquifex aeolicus;
pir∥H69581 Transkriptionsaktivator von Acetoin-Dehydrogenase-Operon acor - Bacillus subtilis;
pir∥I39719 Stickstoffregulatorprotein - Agrobacterium tumefaciens;
pir∥JC5471 Regulatorprotein nifa - Azospirillum lipoferum;
pir∥T08624 wahrscheinlicher Antwortregulator vom ntrc-Typ - Eubacterium acidaminophilum;
sp|P03027|NIFA_KLEPN NIF-SPEZIFISCHES REGULATORPROTEIN;
sp|P09570|NIFA_AZOVI NIF-SPEZIFISCHES REGULATORPROTEIN;
sp|P12627|VNFA_AZOVI STICKSTOFFIXIERUNGSPROTEIN VNFA;
sp|P14375|HYDG_ECOLI TRANSKRIPTIONSREGULATORPROTEIN HYDG;
sp|P21712|YFHA_ECOLI HYPOTHETISCHES 49,1 KD PROTEIN IN GLNB-PURL-INTERGENREGION (ORFXB);
sp|P24426|NIFA_RHILT NIF-SPEZIFISCHES REGULATORPROTEIN;
sp|P25852|HYDG_SALTY TRANSKRIPTIONSREGULATORPROTEIN HYDG;
sp|P27713|NIFA_HERSE NIF-SPEZIFISCHES REGULATORPROTEIN;
sp|P30667|NIFA_AZOBR NIF-SPEZIFISCHES REGULATORPROTEIN;
sp|P38035|RTCR_ECOLI TRANSKRIPTIONSREGULATORPROTEIN RTCR;
sp|P54929|NIFA_AZOLI NIF-SPEZIFISCHES REGULATORPROTEIN;
sp|P56266|NIFA_KLEOX NIF-SPEZIFISCHES REGULATORPROTEIN;
sp|Q06065|ATOC_ECOLI ACETOACETAT-METABOLISMUSREGULATORPROTEIN ATOC (ORNITHIN/ARGININ);
sp|Q46802|YGEV_ECOLI HYPOTHETISCHER, VON SIGMA-54 ABHÄNGIGER TRANSKRIPTIONSREGULATOR IN;
sp|Q53206|NIFA_RHISN NIF-SPEZIFISCHES REGULATORPROTEIN; und
sp|Q9ZIB7|TYRR_ERWHE TRANSKRIPTIONSREGULATORPROTEIN TYRR.

15. Verfahren nach einem der Ansprüche 1 bis 14, wobei der σ54-Transkriptionsaktivator der NifA-Transkriptionsaktivator oder der PspF-Transkriptionsaktivator ist.

16. Verfahren nach einem der Ansprüche 1 bis 14, wobei der Hybrid-σ54-Transkriptionsaktivator NifA ist und Aktivierung, die aus einer NifA-σ54-RNAP-Wechselwirkung resultiert, durch die Coexpression von Wildtyp- oder mutantem NifA verstärkt wird.

17. Verfahren nach Anspruch 16, wobei der Hybrid-σ54-Transkriptionsaktivator NifA von *Azotobacter vinelandii* ist und der Wildtyp- oder mutante NifA ein NifA von *Klebsiella pneumoniae* ist.

18. Verfahren nach einem der vorangegangenen Ansprüche, wobei das Nukleinsäuremolekül eine Bindungsstelle für einen Faktor umfaßt, welcher DNA-Krümmung induziert.

19. Verfahren nach Anspruch 18, wobei der Faktor Integration Host Factor (IHF) ist.

20. Verfahren nach einem der Ansprüche 1 bis 17, wobei das Nukleinsäuremolekül DNA umfaßt, die von sich aus gekrümmt ist.

21. Verfahren nach einem der vorangegangenen Ansprüche, wobei das Nukleinsäuremolekül einen NifA-Promotor von *A. vinelandii* umfaßt, welcher ein Reportergen steuert.

22. Verfahren nach einem der vorangegangenen Ansprüche, wobei das Reportergen aus der Gruppe ausgewählt ist, bestehend aus Metabolismusmarkem, wie dem lac-Operon (IacZ, lacY und lacA), Proteinen, die einen fluoreszenten Phänotyp verleihen, wie GFP, und Proteinen, die Antibiotikaresistenz verleihen, wie Zeo.

23. Verfahren nach einem der vorangegangenen Ansprüche, welches *in vivo* in Bakterien durchgeführt wird.

24. Verfahren nach Anspruch 23, wobei der *in vivo*-Wirt *E. coli* ist.

25. Verfahren nach einem der Ansprüche 1 bis 24, welches *in vitro* durchgeführt wird.

## Revendications

1. Méthode pour détecter une interaction protéine-acide nucléique entre une molécule d'acide nucléique et une molécule de protéine in vitro ou in vivo dans des bactéries, comprenant les étapes consistant :
a) à fournir une ou plusieurs protéines activatrices σ54 hybrides comprenant un domaine de liaison d'acide nucléique hétérologue et un domaine d'activation de transcription de σ54 constitutivement actif ;
b) à fournir une ou plusieurs molécules d'acides nucléiques comprenant un site de liaison pour le domaine de liaison d'acide nucléique et un site de liaison pour l'ARN-polymérase σ54 (RNAP), qui dirige l'expression d'un gène rapporteur et conduit à sa régulation positive en réponse à l'activation par le domaine d'activation de transcription de σ54 ; et
c) à détecter l'expression du gène rapporteur.

2. Méthode suivant la revendication 1, comprenant l'étape consistant à fournir un répertoire de protéines activatrices σ54 hybrides, ledit répertoire comprenant une pluralité de domaines de liaison d'acide nucléique différents.

3. Méthode suivant la revendication 1, comprenant l'étape consistant à fournir un répertoire de molécules d'acides nucléiques hybrides, ledit répertoire comprenant une pluralité de sites de liaison différents pour le domaine de liaison d'acide nucléique.

4. Méthode suivant la revendication 1, comprenant l'étape consistant à fournir un répertoire suivant la revendication 2 et un répertoire suivant la revendication 3.

5. Méthode pour détecter une interaction protéine-protéine in vitro ou in vivo dans des bactéries, comprenant les étapes consistant :
a) à fournir une première protéine hybride comprenant un domaine de liaison d'acide nucléique et une première séquence polypeptidique servant d'appât ;
b) à fournir une seconde protéine hybride comprenant une séquence polypeptidique servant de proie et un domaine d'activation de transcription de σ54 constitutivement actif ;
c) à fournir une molécule d'acide nucléique comprenant un site de liaison pour le domaine de liaison d'acide nucléique et un site de liaison pour σ54 qui dirige l'expression d'un gène rapporteur et conduit à sa régulation positive en réponse à l'activation par le domaine d'activation de transcription de σ54 ;
d) à mettre en incubation les première et seconde protéines hybrides conjointement avec la molécule d'acide nucléique de telle sorte que les séquence polypeptidiques de proie et d'appât puissent se lier, en formant ainsi une protéine hybride comprenant à la fois un domaine de liaison d'acide nucléique et le domaine d'activation de transcription de σ54 ; et
e) à détecter l'expression du gène rapporteur.

6. Méthode suivant la revendication 5, comprenant l'étape consistant à fournir un répertoire de premières protéines hybrides, ledit répertoire comprenant une pluralité de polypeptides servant d'appât.

7. Méthode suivant la revendication 5, comprenant l'étape consistant à fournir un répertoire de secondes protéines hybrides, ledit répertoire comprenant une pluralité de polypeptides servant de proie.

8. Méthode suivant la revendication 5, comprenant l'étape consistant à fournir un répertoire de premières protéines hybrides et un répertoire de secondes protéines hybrides, lesdits répertoires comprenant une pluralité de polypeptides servant d'appât et de polypeptides servant de proie.

9. Méthode pour soumettre à une sélection un répertoire de polypeptides de déformation d'ADN candidats in vivo dans des bactéries ; comprenant les étapes consistant :
a) à fournir un répertoire de facteurs polypeptidiques candidats présentant le potentiel d'induire une déformation de l'ADN ;
b) à fournir une protéine activatrice σ54 comprenant un domaine de liaison d'acide nucléique et un domaine d'activation de transcription de σ54 ;
c) à fournir une molécule d'acide nucléique comprenant un site de liaison pour le domaine de liaison d'acide nucléique et un site de liaison pour la RNAP σ54 qui dirige l'expression d'un gène rapporteur et conduit à sa régulation positive en réponse à l'activation par le domaine d'activation de transcription de σ54 ;
d) à mettre en incubation le répertoire et l'activateur σ54 conjointement avec la molécule d'acide nucléique dans une cellule hôte à facteur d'intégration de l'hôte (IHF), de telle sorte que l'activateur σ54 et la molécule d'acide nucléique puissent interagir, et que la transcription soit activée à partir du site de liaison de RNAP σ54 d'une manière dépendant de la déformation d'ADN par les facteurs polypeptidiques ; et
e) à détecter l'expression du gène rapporteur.

10. Méthode suivant les revendications 1 à 8, dans laquelle les polypeptides peuvent être obtenus par expression dans une cellule hôte bactérienne.

11. Méthode suivant la revendication 10, dans laquelle les polypeptides sont codés dans une ou plusieurs banques de vecteurs d'acides nucléiques.

12. Méthode suivant la revendication 11, dans laquelle une première banque de vecteurs d'acides nucléiques code pour un premier gène chimère, ledit gène comprenant une séquence d'acide nucléique qui code pour un domaine de liaison d'acide nucléique et une séquence d'acide nucléique qui code pour une première protéine d'essai ou un premier fragment de protéine d'essai (d'appât), de telle sorte que la première protéine d'essai soit exprimée en tant qu'une partie d'une protéine hybride avec le domaine de liaison d'acide nucléique.

13. Méthode suivant la revendication 11, dans laquelle une seconde banque de vecteurs d'acides nucléiques code pour un second gène chimère ; ledit gène comprenant une séquence d'acide nucléique qui code pour un domaine d'activation de transcription de σ54 et une seconde protéine d'essai ou un second fragment de protéine d'essai (de proie) dans le vecteur, de telle sorte que la seconde protéine d'essai soit capable d'être exprimée en tant qu'une partie d'une protéine hybride avec le domaine d'activation de transcription.

14. Méthode suivant l'une quelconque des revendications précédentes, dans laquelle l'activateur de transcription de σ54 est choisi dans le groupe consistant en :
dbj|BAA16379.1|(D90877) ACTIVATEUR DE TRANSCRIPTION DE FORMIATE-HYDROGENELYASE ;
emb|CAA26472.1|(X02616) produit du gène Nifa pot. (aa 1-484) [Klebsiella pneumoniae] ;
emb|CAA53584.1| (X75972) anfa [Rhodobacter capsulatus] ;
emb|CAA92413.1| (Z68203) homologue de nifa [Rhizobium sp.] ; emb|CAA93242.1| (Z69251) mopr [Acinetobacter calcoaceticus] ;
emb|CAB53157.1| (X07567) nifa 1 [Rhodobacter capsulatus] ;
emb|CAB56537.1| (AJ249642) régulateur de réponse [Pseudomonas stutzeri] ;
gb|AAA58220.1| (UI89-97) ORF_o532 [Escherichia coli] ;
gb|AAA99303.1| (L43064) protéine régulatrice [Pseudomonas aeruginosa] ;
gb|AAB91397.1|(AF033203) protéine nifail [Rhodobacter capsulatus] ;
gb|AAC05586.1|(AF006075) protéine régulatrice [Bacillus subtilis] ;
gb|AAC37124.1|(L81176) fleq [Pseudomonas aeruginosa] ;
gb|AAC45640.1|(AF010585) activateur sigma 54 putatif [Caulobacter crescentus] ;
gb|AAC46367.1|(AF014113) régulateur de réponse à deux composants [Vibrio cholerae] ;
gb|AAD34591.1|AF145956_1 (AF145956) nifa d'activateur de transcription [Rhodospirillum rubrum] ;
gb|AAD38416.1|(AF155934) nifa [Alcaligenes faecalis] ;
gb|AAF28395.1|(AF069392) flam [Vibrio parahaemolyticus] ;
gb|AAF33506.1|(AF170176) protéine régulatrice de transcription de Salmonella typhimurium ;
gb|AAF61932.1| (AF230804) ActI d'activateur sigma 54
[Myxococcus xanthus] ;
gb|AAF85342.1|AE004061_7 (AE004061) système à deux composants, protéine régulatrice [Xylella fastidiosa] ;
gb|AAF94676.1|(AE004230) régulateur de réponse dépendant de sigma 54 [Vibrio cholerae] ;
gb|AAF95280.1|(AE004286) régulateur de réponse dépendant de sigma 54 [Vibrio cholerae] ;
gb|AAF96095.1|(AE004358) régulateur de transcription dépendant de sigma 54 [Vibrio cholerae] ;
gb|AAG01527.1|AF288483_1 (AF288483) nifa [Azospirillum brasilense] ;
pir||A48291 inhibiteur d'ornithine-décarboxylase - Escherichia coli ;
pir||B49940 homologue du régulateur d'azote I - Escherichia coli ;
pir||C70320 famille nifa des régulateurs de transcription - Aquifex aeolicus ;
pir||C70396 famille ntrc des régulateurs de transcription - Aquifex aeolicus ;
pir||C70454 famille ntrc des régulateurs de transcription - Aquifex aeolicus ;
pir||D70315 famille ntrc des régulateurs de transcription - Aquifex aeolicus ;
pir||H69581 activateur de transcription de l'opéron d'acétoïne-déshydrogénase acor - Bacillus subtilis ;
pir||139719 protéine régulatrice de l'azote - Agrobacterium tumefaciens ;
pir||JC5471 protéine régulatrice nifa - Azospirillum lipoferum ;
pir||T08624 régulateur de réponse du type ntrc probable - Eubacterium acidaminophilum ;
sp|P03027|NIFA_KLEPN PROTEINE REGULATRICE SPECIFIQUE DE NIF ;
sp|P09570|NIFA_AZOVI PROTEINE REGULATRICE SPECIFIQUE DE NIF ;
sp|P12627|VNFA_AZOVI PROTEINE DE FIXATION D'AZOTE VNFA ;
sp|P14375|HYDG-ECOLI PROTEINE REGULATRICE DE TRANSCRIPTION HYDG ;
sp|P21712|YFHA_ECOLI PROTEINE HYPOTHETIQUE DE 49,1 KD DANS LA REGION INTERGENIQUE GLNB-PURL (ORFXB) ;
sp|P24426|NIFA_RHILT PROTEINE REGULATRICE SPECIFIQUE DE NIF ;
sp|P25852|HYDG_SALTY PROTEINE REGULATRICE DE TRANSCRIPTION HYDG ;
sp|P27713|NIFA_IIERSE PROTEINE REGULATRICE SPECIFIQUE DE NIF ;
sp|P30667|NIFA_AZOBR PROTEINE REGULATRICE SPECIFIQUE DE NIF ;
sp|P38035|RTCR_ECOLI PROTEINE REGULATRICE DE TRANSCRIPTION RTCR ;
sp|P54929|NIFA_AZOLI PROTEINE REGULATRICE SPECIFIQUE DE NIF ;
sp|P56266|NIFA_KLEOX PROTEINE REGULATRICE SPECIFIQUE DE NIF ;
sp|Q06065|ATOC_ECOLI PROTEINE REGULATRICE DU METABOLISME DE L'ACETOACETATE ATOC (ORNITHINE/ARGININE) ;
sp|Q46802|YGEV_ECOLI REGULATEUR DE TRANSCRIPTION HYPOTHETIQUE DEPENDANT DE SIGMA 54 IN ;
sp|Q53206|NIFA_RHISN PROTEINE REGULATRICE SPECIFIQUE DE NIF ; et
sp|Q9ZIB7|TYRR_ERWHE PROTEINE REGULATRICE DE TRANSCRIPTION TYRR.

15. Méthode suivant l'une quelconque des revendications 1 à 14, dans laquelle l'activateur de transcription de σ54 est l'activateur de transcription NifA ou l'activateur de transcription PspF.

16. Méthode suivant l'une quelconque des revendications 1 à 14, dans laquelle l'activateur de transcription de σ54 hybride est l'activateur NifA et l'activation résultant de l'interaction NifA-RNAP σ54 est accrue par la coexpression du NifA de type sauvage ou mutant.

17. Méthode suivant la revendication 16, dans laquelle l'activateur de transcription de σ54 est l'activateur NifA provenant d'*Azotobacter vinelandii*, et le NifA de type sauvage ou mutant est le NifA provenant de *Klebsiella pneumoniae*.

18. Méthode suivant l'une quelconque des revendications précédentes, dans laquelle la molécule d'acide nucléique comprend un site de liaison pour un facteur qui induit une déformation de l'ADN.

19. Méthode suivant la revendication 18, dans laquelle le facteur est le facteur d'intégration de l'hôte (IHF).

20. Méthode suivant l'une quelconque des revendications 1 à 17, dans laquelle la molécule d'acide nucléique comprend un ADN qui est intrinsèquement déformé.

21. Méthode suivant l'une quelconque des revendications précédentes, dans laquelle la molécule d'acide nucléique comprend un promoteur NifH provenant d'*A*. *vinelandii* commandant un gène rapporteur.

22. Méthode suivant l'une quelconque des revendications précédentes, dans laquelle le gène rapporteur est choisi dans le groupe consistant en un marqueur métabolique tel que l'opéron lac (lacZ, lacY et lacA) ; des protéines conférant un phénotype fluorescent, telles que GFP ; des protéines conférant une résistance à un antibiotique, telles que Zeo.

23. Méthode suivant l'une quelconque des revendications précédentes, qui est mise en oeuvre *in vivo* dans des bactéries.

24. Méthode suivant la revendication 23, dans laquelle l'hôte *in vivo* est *E. coli*.

25. Méthode suivant l'une quelconque des revendications 1 à 24, qui est mise en oeuvre *in vitro.*
